(19) Europäisches Patentamt / European Patent Office / Office européen des brevets

(11) **EP 4 397 668 A1**

(12) **EUROPEAN PATENT APPLICATION**
published in accordance with Art. 153(4) EPC


(43) Date of publication:
**10.07.2024 Bulletin 2024/28**

(21) Application number: **22865053.7**

(22) Date of filing: **31.08.2022**

(51) International Patent Classification (IPC):
***C07H 21/00*** *(2006.01)* ***C12N 15/113*** *(2010.01)*
***A61K 31/7088*** *(2006.01)*

(52) Cooperative Patent Classification (CPC):
**A61K 31/7088; A61K 48/00; C07H 1/00; C07H 19/16; C07H 21/00; C12N 15/11; C12N 15/113**

(86) International application number:
**PCT/KR2022/013062**

(87) International publication number:
**WO 2023/033551 (09.03.2023 Gazette 2023/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2021 KR 20210115942**



(71) Applicant: **Hanmi Fine Chemical Co., Ltd.**
**Siheung-Si, Gyeonggi-do 15093 (KR)**

(72) Inventors:
- **CHOI, Bo Sung**
**Siheung-si, Gyeonggi-do 15011 (KR)**
- **KIM, Na Ri**
**Incheon 21673 (KR)**
- **NOH, Jin Mi**
**Siheung-si, Gyeonggi-do 15093 (KR)**
- **JUNG, Yong Gyu**
**Siheung-si, Gyeonggi-do 15093 (KR)**
- **MIN, Su Hyeon**
**Siheung-si, Gyeonggi-do 15093 (KR)**

(74) Representative: **Brevalex**
**Tour Trinity**
**1 B Place de la Défense**
**92400 Courbevoie (FR)**


(54) **MRNA CAP ANALOGUE AND USE THEREOF**


(57) One aspect relates to a compound of Formula 1, a method of preparing an intermediate thereof, a cap analog including the same, an mRNA 5'-capped with the cap analog, a method of preparing mRNA using the cap analog, use for using the cap analog in preparing mRNA, and a pharmaceutical composition for expressing a target peptide or protein, including the mRNA 5'-capped with the cap analog, and the like.


EP 4 397 668 A1

## Description

Technical Field

The present disclosure relates to cap analogs and methods of protein expression using the same, and more specifically to a cap analog, a 5' capped mRNA including the cap analog, a pharmaceutical composition for expressing a target peptide or protein including the 5' capped mRNA, and a method of producing the target peptide or protein by using the 5' capped mRNA, etc.

Background Art

In order to express a functionally important specific protein from DNA genetic information, a transcription process is required in the nucleus in which messenger RNA (mRNA) with a complementary sequence is synthesized from a DNA template strand. The synthesized mRNA is then transported out of the nucleus and undergoes a translation process in the cytoplasm, where it is used by ribosomes to synthesize specific proteins.

The mRNA undergoes a post-transcriptional maturation process to efficiently translate the encoding protein. During this process, the process in which 7-methylguanosine ($^{7m}$G) is connected to the 5'-end of the mRNA molecule by bonding through a triphosphate group by the 5'-end is called the 5' capping process. The 5' cap structure (referred to as the 5' cap 0 mRNA structure) may protect the 5' end of the mRNA from biodegradation by 5' exonucleases and affects the translocation of the mRNA from the nucleus to the cytoplasm. In particular, the 5' cap structure is recognized by eukaryotic translation initiation factor 4E (eIF4E), which plays an important role in protein expression by forming a translation initiation complex.

In order for this capped mRNA to be clinically applicable, it must have a structure (Cap1 mRNA) in which the 2'hydroxyl group of the first nucleotide linked to 7-methylguanosine through a 5'-triphosphate chain is methylated. When applying the unmethylated CapO structure, MDA5 (an intracellular sensor protein), which recognizes the CapO structure, recognizes the 2' hydroxyl group when an exogenous mRNA with a CapO structure is introduced into the body and induces an immune-inflammatory response, which impairs the bonding of the mRNA to eIF4E and thus inhibits protein expression. On the other hand, exogenous Cap1 mRNA with 2'OH methylated does not induce an immune response when introduced into the body because it is not recognized by MDA5, and therefore may induce relatively high protein expression, making it suitable for clinical use.

Recently, in vitro mRNA synthesis research has been actively conducted based on the structural characteristics of the 5' end of active mRNA described above, and in particular, 5' capping methods are also continuously being developed. In in vitro mRNA synthesis, 5' capping may be broadly divided into two methods. The conventional capping method is a method of obtaining a structure (cap 0) in which the 5' end is capped with 7-methylguanosine by treating the enzyme involved in 5' capping, that is, a mixture called "capping enzyme", after in vitro transcription. In order to create a cap1 or cap2 mRNA structure, the mRNA must be additionally treated with an enzyme capable of methylating the 2' hydroxyl group of the nucleotide, such as vaccinia mRNA (nucleoside-2'-O) methyltransferase. This conventional capping method has the disadvantage of being expensive to prepare due to the enzyme cost and number of treatments, and is difficult to control the enzyme reaction.

To overcome such disadvantages of the conventional capping method, a co-transcriptional capping method is being researched. The co-transcriptional capping method refers to a method of synthesizing mRNA in which a cap analog constitutes the 5' end by simultaneously introducing a chemically synthesized cap analog during in vitro mRNA synthesis. The co-transcriptional capping method has evolved from the first generation of dimeric cap analogs, mCAP ($^{7m}$G(5')ppp(5')G), to the third-generation of trinucleotide cap analogs, Cleancap® ($^{7m}G_{(3'OMe)}$pppN$_{(2'OMe)}$pN) (U.S. Patent No. 10,913,768) from TriLink, with each generation improving upon the shortcomings of the previous generation. While mCAP has a lower production cost compared to the conventional capping method, it is limited to the 5' cap 0 mRNA structure and has the disadvantage of acting competitively with guanosine triphosphate (GTP) when mCAP forms base pairing on the DNA template. In order to perform base pairing on the DNA template more favorably than GTP, it is necessary to add 4 times to 10 times the amount of GTP used, which is a factor in increasing the production cost. In addition, mCAP in the form of a dinucleotide, may undergo reverse bonding, resulting in bidirectional transcription initiation, which reduces the efficiency of forward mRNA synthesis in vitro.

To address this problem of reverse transcription initiation, a second-generation cap analog, Anti-reverse cap analog (aka ARCA), was developed (US Patent 7,074,596). ARCA is a dimeric cap analog with the structure $^{7m}G_{(3'OMe)}$pppG or $^{7m}G_{(2'OMe)}$pppG in which either the 2' or 3' hydroxyl group of 7-methylguanosine is substituted by a methoxy group in the dimeric $^{7m}$GpppG, which prevents reverse bonding of the cap analog and thus completely blocks reverse transcription initiation RNA (see 9:1108-1122 (2003)). In addition, it was confirmed that the ARCA, in which either the 2' or 3' hydroxyl group was chemically modified, had increased protein expression efficiency compared to the cap analog in which both the 2' and 3' were hydroxyl groups. Either of chemical modification of the 2' hydroxyl group and 3' hydroxyl

group showed equivalent levels of protein expression efficiency regardless of position. However, the second-generation cap analog did not solve the problem of competitive base pairing with GTP. Although production of mRNA in the reverse direction was inhibited, there were still problems with the generation of mRNA impurities with the pppG 5' end (called uncapped mRNA) or the introduction of an excessive amount of cap analog compared to GTP. In addition, in-vitro mRNA synthesis using ARCA has the disadvantage of inducing an innate immune response due to the inability to synthesize a cap 1 structure.

In the case of Cleancap®, a third-generation cap analog developed by TriLink, it has a structure of at least trinucleotides, and the Cap1 structure ($^{7m}GpppN_{(2'OMe)}pN$) may be synthesized in vitro without using enzymes. In addition, because it has at least one more base than GTP, it may hydrogen bond more strongly with the DNA template strand at the start of transcription to perform base pairing, and thus may reduce the input amount of cap analog during in vitro mRNA synthesis. The currently commercialized Cleancap®, $^{7m}G_{(3'OMe)}pppA_{(2'OMe)}pG$, is a trinucleotide cap that methylates the 3' hydroxyl group of 7-methylguanosine, and has shown improved protein expression efficiency over the 2', 3' hydroxyl cap, $^{7m}GpppA_{(2'OMe)}pG$.

A commercialized mRNA vaccine based on the Cleancap structure described above ($^{7m}G_{(3'OMe)}pppA_{(2'OMe)}pG$) includes BNT162b2 from Pfizer/BioNTech. BNT162b2 was developed to respond to infectious diseases caused by severe acute respiratory syndrome coronavirus 2 (SARS-CoV-2), an RNA virus that caused a global pandemic. BNT162b2, which is manufactured using Cleancap as a key raw material, had difficulty meeting the demand to respond to the global Covid-19 pandemic, and at one time experienced a raw material supply shortage. In order to mass-produce mRNA vaccines that may adequately respond to highly transmissible RNA virus infectious diseases, rapid and economical production and supply of raw materials are required, among them, a variety of additional research and development must be conducted to efficiently manufacture and quickly supply cap analogs, which account for the highest production costs.

Looking at the manufacturing process of TriLink's currently commercialized trinucleotide cap analog, it is largely divided into six major processes as shown below (US Patent 10,913,768).

[Reaction 1-1] $G_{(3'OMe)} \rightarrow pG_{(3'OMe)} \rightarrow Im\text{-}pG_{(3'OMe)} \rightarrow ppG_{(3'OMe)} \rightarrow pp^{7m}G_{(3'OMe)} \rightarrow Im\text{-}pp^{7m}G_{(3'OMe)} + pN_{(2'OMe)}pN \rightarrow {}^{7m}G_{(3'OMe)}pppN_{(2'OMe)}pN$

In Reaction 1-1, wherein G refers to guanosine, $^{7m}G$ refers to 7-methylguanosine, N refers to any one of adenosine, cytidine, guanosine, and uridine, p refers to $-P(=O)O_2^-$, Me refers to methyl, and Im refers to an imidazolide. Moreover, looking at the details, if a process of synthesizing $pN_{(2'OMe)}pN$, the reactant used in the final manufacturing process, is included, a long process of at least 10 steps must take place.

In particular, when mass-producing TriLink's cap analogs, the main factor that increases the production cost and synthesis period in the entire manufacturing process is the purification processes using ion exchange chromatography (mainly DEAE resin) that must be performed between steps of the manufacturing process. After purifying the intermediate for each manufacturing process, a large amount of buffer solution (mainly triethylammonium bicarbonate (TEAB) buffer) used in column purification must be distilled to proceed with the next process in an organic solvent. Since purification must be performed at each step of generation process for $pG_{(3'OMe)}$, $ppG_{(3'OMe)}$, $pp^{7m}G_{(3'Ome)}$, $pN_{(2'OMe)}pN$, and $^{7m}G_{(3'OMe)}pppN_{(2'OMe)}pN$, in the entire manufacturing process, a total of 5 ion exchange column purifications are required. In particular, for the production of $G_{(3'OMe)}$, it is necessary to selectively perform monoetherification of only the 3'-OH of the three hydroxyl groups of the guanosine nucleoside, and it is also necessary to purify only the 3'-monoetherified product among the products, resulting in a problem wherein the manufacturing cost of $pG_{(3'OMe)}$, the starting substance, is high. This lengthy process, column purification, and distillation of the buffer solution increases the time and cost required for the preparation of cap analogs, lowering their economic feasibility.

Furthermore, in order to prepare 3'-methoxy guanosine (G(3'OMe)), the starting substance for the preparation of ARCA and TriLink cap analog, only the monoether substances substituted with 3'methoxy may be isolated by inducing a methylation reaction on the 2' and 3' hydroxyl groups. As an example, looking at the process of preparation of 3'-methoxy guanosine described in the ARCA invention, it is divided into three steps as shown below (RNA 9:1108-1122 (2003)).

[Reaction 1-2]

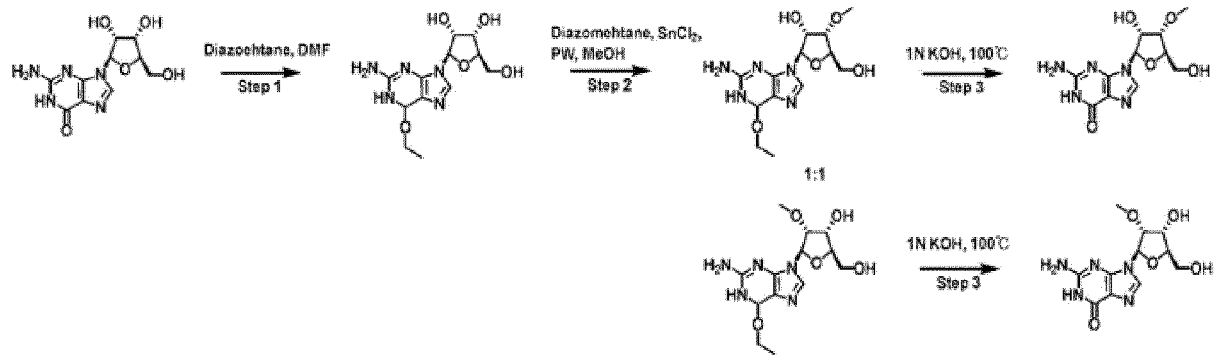


Flash chromatography or Dowex 1 × 2 ion exchange chromatography must be performed at each step, and the yield of each step is low, making mass-production difficult and increasing production costs. Recently, research and development has been conducted to increase the efficiency of 3'-methoxy guanosine synthesis, but the manufacturing processes are prolonged and there are still difficulties in isolating the monoether substances substituted with 3'-methoxy. This is a major factor in increasing the raw material cost for synthesizing cap analogs.

[PRIOR ART]

[PATENT DOCUMENTS]

(Patent Document 001) US 7,074,596
(Patent Document 002) US 10,913,768

[NON-PATENT DOCUMENTS]

(Non-Patent Document 001) RNA 9:1108-1122 (2003)

Disclosure

Technical Problem

One aspect provides a cap analog that may increase the efficiency of in-vitro synthesis of a 5'-capped mRNA molecule, and may increase the efficiency of protein expression of the capped mRNA, and enable economic production of the cap analog itself.

Another aspect provides a method of preparing an intermediate for preparing the cap analog.

Another aspect provides an mRNA 5'-capped with the cap analog.

Another aspect provides a method of preparing an mRNA utilizing the cap analog.

Another aspect provides a composition or kit for preparing 5' capped mRNA including the cap analog.

Another aspect provides a pharmaceutical composition for the expression of a target peptide or protein, including the cap analog.

Another aspect provides a cell containing an mRNA 5'-capped with the cap analog.

Another aspect provides a cell containing a protein or peptide translated from an mRNA 5'-capped with the cap analog.

Technical Solution

One aspect provides a compound of Formula 1 or a pharmaceutically acceptable salt thereof:

[Formula 1]

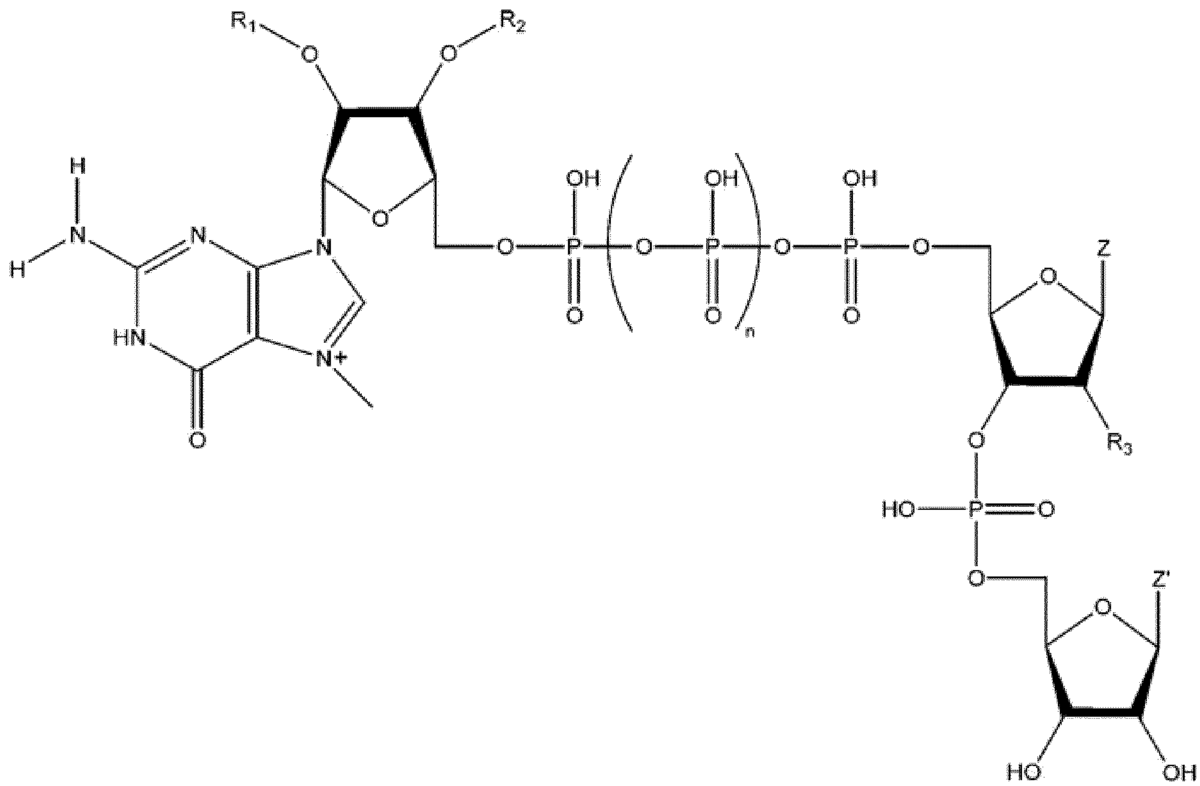

wherein

n = 0, 1, or 2;
$R_1$ and $R_2$ are each independently H or C(=O)-R', wherein at least one of $R_1$ and $R_2$ is C(=O)-R', wherein R' is $C_1$ to $C_6$alkyl or $C_1$ to $C_6$alkoxy,
$R_3$ is a methoxy group;
Z and Z' are each independently a natural nitrogen base.

Another aspect provides an aqueous composition including a compound of Formula 1A and Formula 1B in a molar ratio of 0.65$\pm$0.05 : 1:

[Formula 1A]

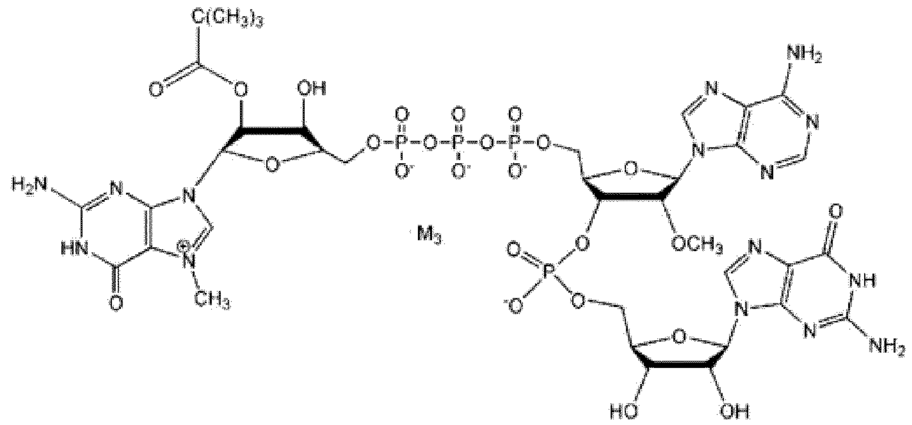

[Formula 1B]

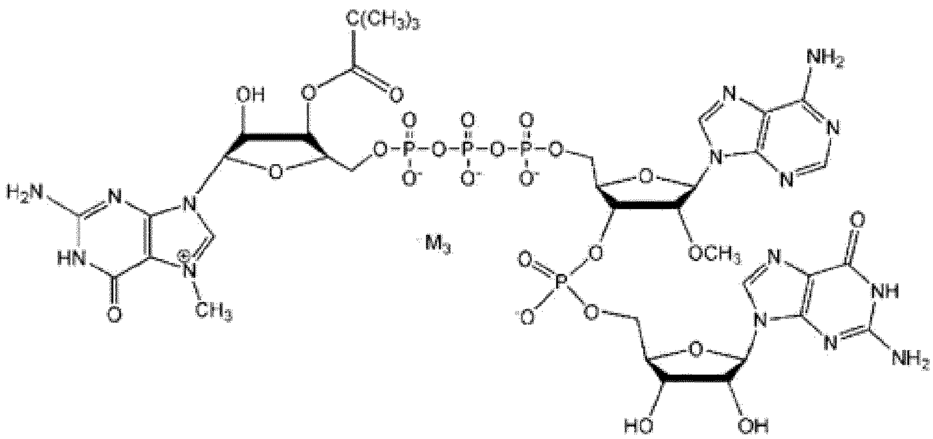

Here, each M of $M_3$ independently is either not present, or is a monovalent cation selected from the group consisting of $Na^+$, $Li^+$, $NH_4^+$, and $K^+$, or a divalent cation selected from the group consisting of $Mg^{2+}$, $Zn^{2+}$, and $Ca^{2+}$, wherein $M_3$ is selected such that the compound is electrically neutral.

Another aspect provides a cap analog including the compound of Formula 1 above.

Another aspect provides an mRNA 5'-capped with the cap analog.

Another aspect provides a method of preparing an mRNA, including incorporating the cap analog during synthesis of the mRNA.

Another aspect provides a composition or kit for preparing 5' capped mRNA including the cap analog.

Another aspect provides a pharmaceutical composition for expressing a desired peptide or protein, including an mRNA 5'-capped with the cap analog and a pharmaceutically acceptable carrier.

Another aspect provides a cell including an mRNA 5'-capped with the cap analog.

Another aspect provides a cell containing a protein or peptide translated from an mRNA 5'-capped with the cap analog.

Another aspect provides, a method of preparing a compound of Formula 6 or a salt thereof, comprising steps of

preparing a compound of formula 4 or a salt thereof by reacting a compound of Formula 2 below or a salt thereof with a compound of Formula 3 below under alkaline conditions in the aqueous phase; and
reacting the compound of Formula 4 or its salt with the compound of Formula 5 under pH 1.5 to 4.5 conditions with an *In situ* method:

[Formula 2]

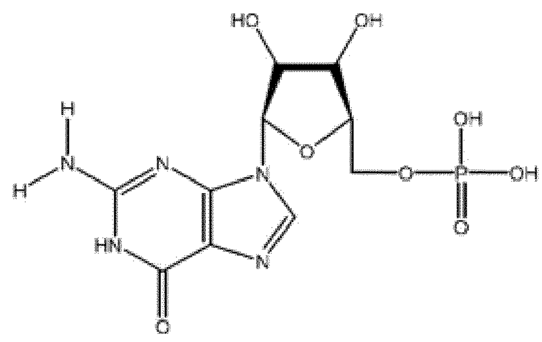

[Formula 3]

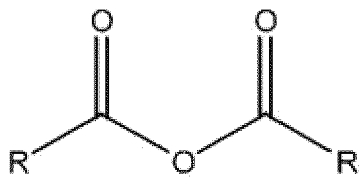

[Formula 4]

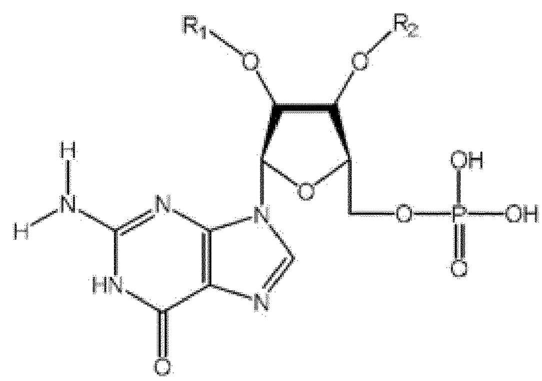

[Formula 5]

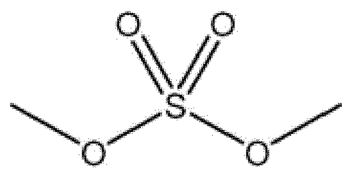

[Formula 6]

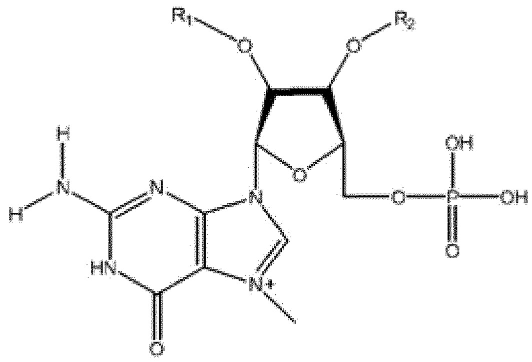

in the above formula, R refers to $C_1$ to $C_6$alkyl or $C_1$ to $C_6$alkoxy, and $R_1$ and $R_2$ are each independently H or C(=O)-R', wherein one of $R_1$ and $R_2$ is H and the other is C(=O)-R.

## Advantageous Effects

A cap analog according to an aspect, such as the trinucleotide cap analog ($^{7m}G_{(3'OMe)}pppA_{(2'OMe)}pG$) of TriLink, do not require selective introduction of protecting groups at specific positions and do not require purification of the selectively introduced compounds, does not require expensive starting substances such as 3'-methoxy guanosine, and has the advantage of being able to simplify the manufacturing process, thereby improving the efficiency and economy of synthesis, while enabling an excellent level of expression efficiency of the capped mRNA when capping mRNA.

As such, the cap analog has excellent advantages in terms of function and production cost, and mRNA including the cap analog of the present disclosure may be very useful in treating or preventing diseases in mammals, including humans.

## Description of Drawings

FIG. 1 is a graph illustrating the result of $^1H$ NMR measurement after preparation of an intermediate compound (Example 8) according to an embodiment of the present disclosure.

FIG. 2 is a graph illustrating the result of $^1H$ NMR measurement after preparation of an intermediate compound (Example 9) according to an embodiment of the present disclosure.

FIG. 3 is a graph illustrating the result of $^1H$ NMR measurement after preparing a compound (compound of Example 16) according to an embodiment of the present disclosure.

FIG. 4 is a graph illustrating the relative in-vitro transcription (IVT) yield (%) of a compound of Example 15 or 16 compared to $^{7m}G_{(3'OMe)}pppA_{(2'OMe)}pG$ after measuring the IVT yield by using a compound (compound of Example 15 or Example 16) according to an embodiment of the present disclosure or $^{7m}G_{(3'OMe)}pppA_{(2'OMe)}pG$ as a cap analog.

FIG. 5 illustrates the translation activity of mRNA produced by the IVT reaction of each of a compound (compound of Example 15 or Example 16) according to an embodiment of the present disclosure or $^{7m}G_{(3'OMe)}pppA_{(2'OMe)}pG$ measured by transfecting the corresponding mRNA into a HeLa cell line, which shows the results as the amount of luciferase expression.

FIG. 6 illustrates the translation activity of mRNA produced by the IVT reaction of each of a compound (compound of Example 15 or Example 16) according to an embodiment of the present disclosure or $^{7m}G_{(3'OMe)}pppA_{(2'OMe)}pG$ as measured at 24 hours after transfection of the corresponding mRNA into the HeLa cell line, which shows the results as the amount of luciferase expression.

## Mode for Invention

Hereinafter, the present disclosure is described in more detail.

All technical terms used in the present disclosure, unless otherwise defined, are used as commonly understood by one of ordinary skill in the relevant field of the present disclosure. In addition, while suitable methods or samples are described herein, similar or equivalent methods are also included within the scope of the present disclosure. Furthermore,

figures given herein shall be deemed to include the meaning of "about" even if not specified. The contents of all publications incorporated by reference herein are hereby incorporated by reference in their entirety.

Definitions of Terms

As used herein, the following terms shall have the meanings as defined below, unless otherwise indicated.

In the context of a complex of a cap analog and a DNA template, the terms "complementary" or "complementarity" as used herein refers to standard Watson/Crick base pairing rules. For example, the sequence "5'-A-G-T-C-3"' is complementary to the sequence "3'-T-C-A-G-5"'. Complementarity does not have to be perfect; a duplicate may contain mismatched base pairs, or modified or non-matched nucleotides. One of ordinary skill in the art may determine duplex stability by experimentally considering numerous variables, including for example, the length of oligonucleotide, the base composition and sequence of oligonucleotide, the incidence of mismatched base pairs, ionic strength, components of the hybridization buffer, and reaction conditions.

Complementarity may be "complete" or "total" if all of the nucleotide bases of the two nucleic acid strands match according to recognized base pairing rules, may be "partial" if only some of the nucleotide bases of the cap analog and DNA target match according to recognized base pairing rules, or may be "absent" if none of the nucleotide bases of the two nucleic acid strands match according to recognized base pairing rules.

As used herein, the term "nitrogenous base" includes all naturally occurring nitrogenous bases. The most common base rings found in naturally occurring nitrogenous bases are purine and pyrimidine rings. Naturally occurring purine rings include, for example, adenine, guanine, and N6-methyladenine. Naturally occurring pyrimidine rings include, for example, cytosine, thymine, 5-methylcytosine, and uracil. Naturally occurring nucleoside include, but are not limited to, for example, adenosine, guanosine, cytidine, thymidine, uridine, inosine, 7-methylguanosine, or a ribo, 2'-O-methyl, or 2'-deoxyribo derivative of uridine.

The term "$C_{1-n}$alkyl" refers to a linear or branched saturated hydrocarbon radical chain of 1 to n carbon atoms. Specific examples may include, but are not limited to, methyl, ethyl, n-propyl, isopropyl, n-butyl, isobutyl, t-butyl, n-pentyl, isopentyl, and n-hexyl, etc.

The term "C1-nalkoxy" refers to a linear or branched, saturated hydrocarbon radical chain of 1 to n carbon atoms bonded to oxygen. Specific examples may include, but are not limited to, methoxy, ethoxy, propoxy, isobutoxy, n-butoxy, sec-butoxy, t-butoxy, pentoxy, hexoxy, etc.

In the process of synthesizing substances, the "*In situ* method" refers to performing the next process of the reaction in the original container without additional purification of the obtained product.

One aspect relates to a compound that has the structure of Formula 1 below:

[Formula 1]

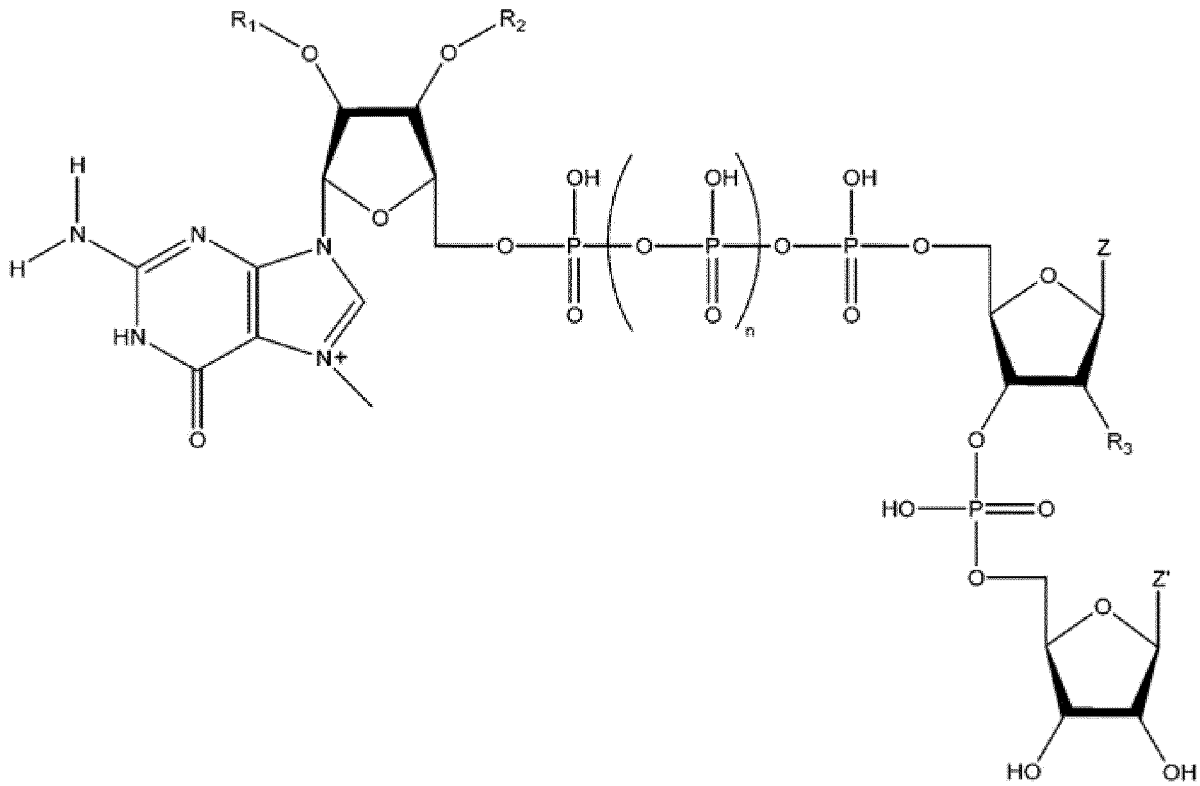

wherein

n = 0, 1, or 2;
$R_1$ and $R_2$ are each independently H or C(=O)-R', wherein at least one of $R_1$ and $R_2$ is C(=O)-R', wherein R' is $C_1$ to $C_6$alkyl or $C_1$ to $C_6$alkoxy,
$R_3$ is a methoxy group;
Z and Z' are each independently a natural nitrogenous base.

The pharmaceutically acceptable salt refers to a salt commonly used in the pharmaceutical field, and may specifically be a base addition salt. The salts include, for example, a monovalent metal salt, a divalent metal salt, an amine salt, or an amino acid salt. The monovalent metal salts include, but are not limited to, Na, Li, or K salts, the divalent metal salt include Ca, Zn, or Mg salts, and the amine salt includes trimethylamine, triethylamine, ammonia, pyridine, or picoline salt, etc., and the amino acid salt include arginine, lysine, or histidine salt, etc.

The pharmaceutically acceptable salt may be in the form of a salt that allows the compound of Formula 1 to form a stable, electrically neutral form in an aqueous liquid phase.

A compound of Formula 1 or a pharmaceutically acceptable salt thereof may be, for example, a compound of Formula 1A below:

[Formula 1A]

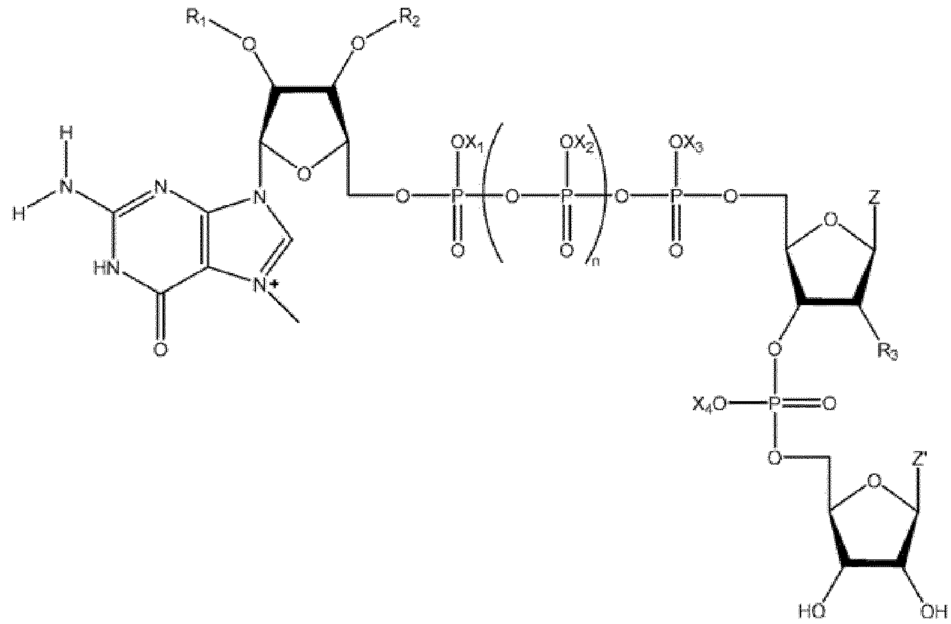

wherein

n = 0, 1, or 2;
X1, X2, X3, and X4 are each independently absent, or are a monovalent cation, a divalent cation, or a combination thereof, wherein X1, X2, X3, and X4 are selected such that the compound of Formula 1A is electrically neutral;
$R_1$ and $R_2$ are each independently H or C(=O)-R', wherein at least one of $R_1$ and $R_2$ is C(=O)-R', wherein R' is $C_1$ to $C_6$alkyl or $C_1$ to $C_6$alkoxy
$R_3$ is a methoxy group;
Z and Z' are each independently a natural nitrogenous base.

In an embodiment, the monovalent cation is selected from the group consisting of $Na^+$, $Li^+$, $NH_4^+$, and $K^+$, and the divalent cation is selected from the group consisting of $Mg^{2+}$, $Zn^{2+}$, and $Ca^{2+}$.
In an embodiment, the R' is $C_2$-$C_6$alkyl or $C_2$-$C_6$alkoxy.
In an embodiment, R' is methyl, ethyl, isopropyl, isopropoxy, t-butyl, or t-butoxy.
In an embodiment, Z and Z' in Formula 1 are each independently in the form of a natural purine or pyrimidine base moiety. More specifically, Z and Z' may each be independently selected from the group consisting of guanine, adenine, cytosine, thymine, and uracil. In an embodiment, Z and Z' are adenine and guanine, respectively.
In an embodiment, when only one of R1 and R2 in Formula 1 has C(=O)-R', the structural isomers present at the 2' or 3' position of guanosine may exist in a form of a mixture in dynamic equilibrium with each other. The ratio of each structural isomer forming dynamic equilibrium (that is, an isomer with -OC(=O)R' at the 2' position: an isomer with -OC(=O)R' at the 3' position) may vary depending on the structure of the specific compound and may also vary depending on the surrounding environment (for example: temperature, pH).
In an embodiment, the compound of Formula 1 is a compound selected from the group consisting of the compounds listed in Table 1 below, or a pharmaceutically acceptable salt thereof:

[Table 1]

| Example | Structural Formula | Compound Name |
|---|---|---|
| 15 | 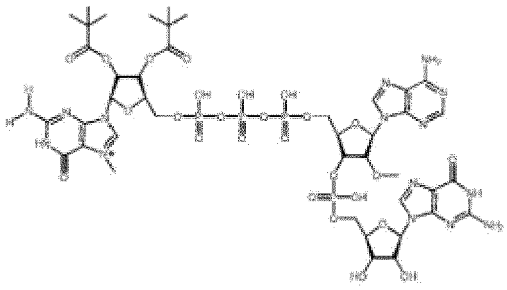 | $^{7m}G_{(2',3'\ \text{Pivaloyl})}pppA_{(2'\ \text{OMe})}pG$ |
| 16 | 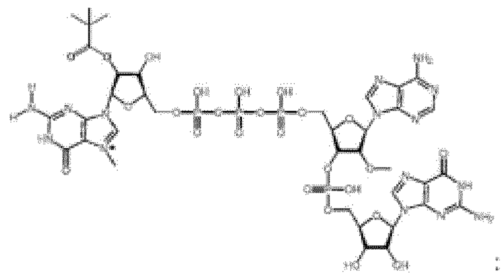 : 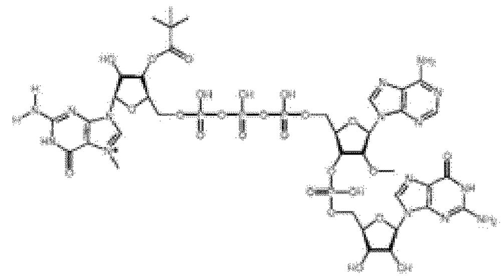 | Mixture in molar ratio of $^{7m}G_{(2'\ \text{monopivaloyl})}pppA_{(2'\ \text{OMe})}pG$ : $^{7m}G_{(3'\ \text{monopivaloyl})}pppA_{(2'\ \text{OMe})}pG = 0.65 \pm 0.05:1$ (in $H_20$, 25 °C$\pm$5, pH 1.0 ~ 8.0) |
| 17 | 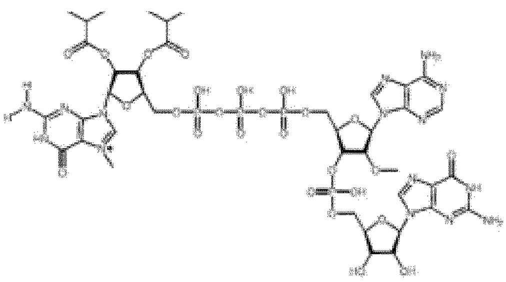 | $^{7m}G_{(2',3'\ \text{Isobutyryl})}pppA_{(2'\text{OMe})}pG$ |

(continued)

| Example | Structural Formula | Compound Name |
|---|---|---|
| 18 | 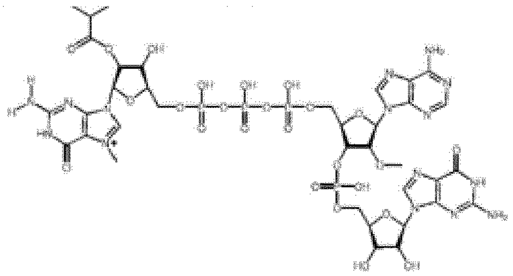 : 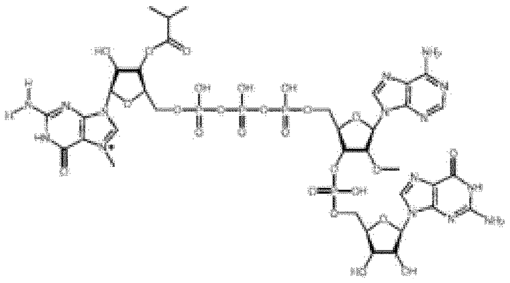 | Mixture in molar ratio of $^{7m}G_{(2' \text{ monoisobutyryl})}pppA_{(2' \text{ OMe})}p$ : $^{7m}G_{(3' \text{ monoisobutyryl})}pppA_{(2' \text{ OMe})}pG$ = 0.4510.25:1 (in $H_20$, 25 °C±5, pH 1.0 ~ 8.0) |
| 19 | 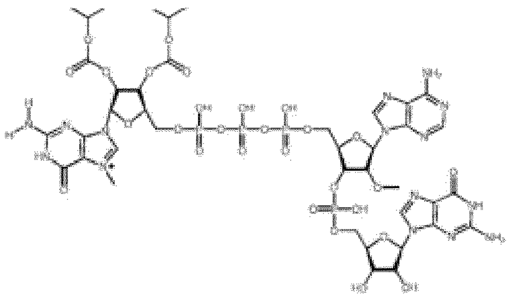 | $^{7m}G_{(2',3' \text{ Isopropoxy carbonyl})}pppA_{(2' \text{ OMe})}pG$ |
| 20 | 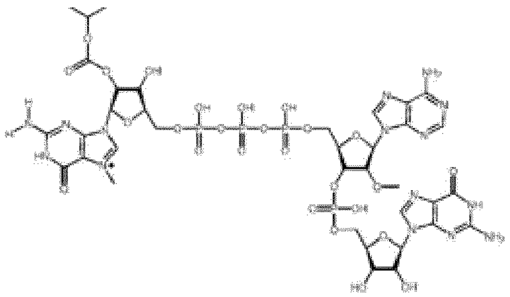 | Mixture in molar ratio of $^{7m}G_{(2' \text{ monatsopropoxy carbonyl})}ppA_{(2' \text{ OMe})}p$ : $^{7m}G_{(2' \text{ monoisopropoxy carbonyl})}pppA_{(2' \text{ OMe})}pG$ = 0.45±0.25:1 (in $H_20$, 25 °C±5, pH 1.0 ~ 80) |
| | 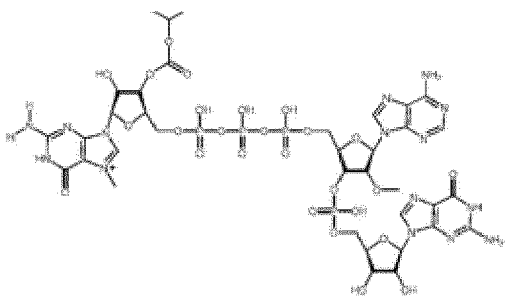 | |

(continued)

| Example | Structural Formula | Compound Name |
|---|---|---|
| 21 | 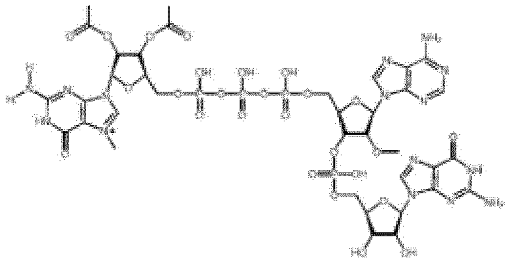 | $^{7m}G_{(2',3'Acetyl)}pppA_{(2'OMe)}pG$ |
| 22 | 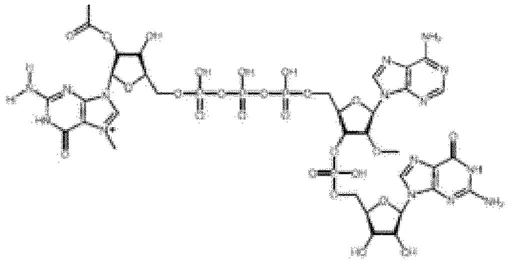 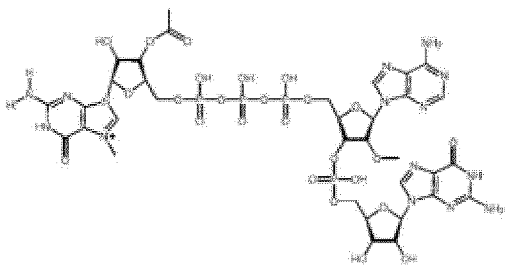 | Mixture in molar ratio of $^{7m}G_{(2'\ monoscetyl)}pppA_{(2'\ OMe)}pG$ : $^{7m}G_{(3'\ monoscetyl)}pppA_{(2'\ OMe)}pG$ = 0.45±0.25:1 (in $H_20$, 25 °C±5, pH 1.0 ~ 8.0) |

In the compound name, G refers to guanosine, $^{7m}G$ refers to 7-methylguanosine, A refers to adenosine, p refers to -P(=O)(OH)O-, and Me refers to methyl. Another aspect provides an aqueous composition comprising compounds of Formula 1a and Formula 1b below in a molar ratio of 0.65±0.05:1:

[Formula 1A]

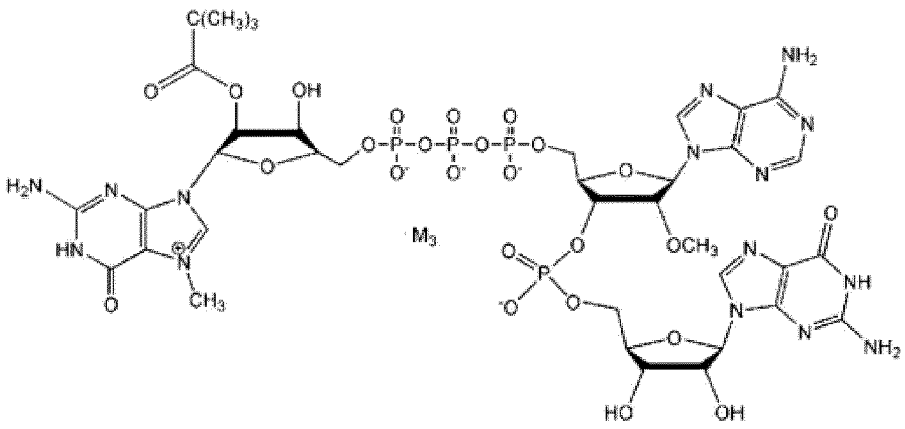

[Formula 1B]

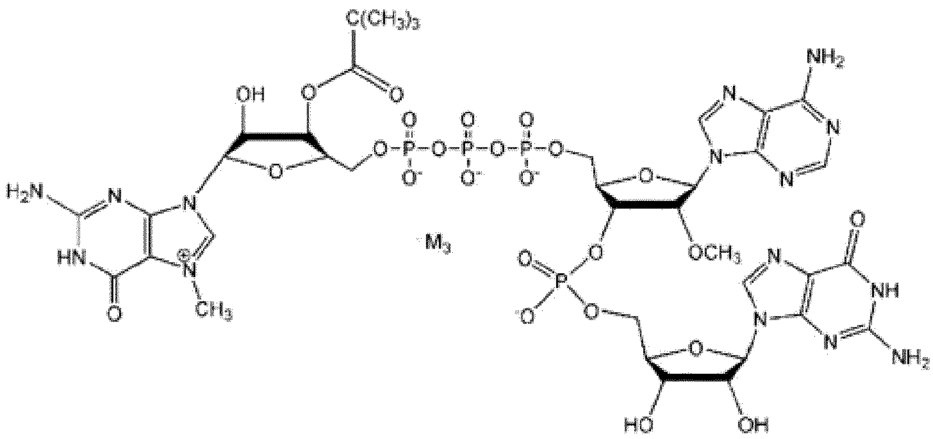

Here, each M of $M_3$ is independently either not present , or is a monovalent cation selected from the group consisting of $Na^+$, Li+, $NH_4^+$, and K+, or a divalent cation selected from the group consisting of $Mg^{2+}$, $Zn^{2+}$, and $Ca^{2+}$, wherein $M_3$ is selected such that the compound is electrically neutral. For example, $M_3$ may indicate that all three M's are $NH_4^+$, or that one M among $M_3$ is $Ca^{2+}$, one M is $K^+$, and one M is absent. In an embodiment, each M of the $M_3$ is all $Na^+$.

In an embodiment, the pH of the aqueous composition is from 1.0 to 8.0.

The compounds of Formula 1A and Formula 1B may exist, for example, as a mixture in an aqueous composition at 25 °C $\pm$ 5 and pH 1.0 to 8.0 at a molar ratio of 0.65 $\pm$ 0.05:1.

The compounds of Formula 1 may be prepared, for example, according to the methods illustrated in the following embodiment. One of ordinary skill in the art may appropriately modify the methods illustrated in the examples below to prepare the above compounds by modifying the reaction conditions, reaction sequence, and reaction compounds.

The preparation method exemplified in the Examples of the compound of Formula 1 above may be briefly indicated by the following abbreviation.

[Reaction 2] pG $\rightarrow$ $p^{7m}G_{(2'\text{ and/or }3'OC(=O)R')}$ $\rightarrow$ $pp^{7m}G_{(2'\text{ and/or }3'OC(=O)R')}$ $\rightarrow$ Im-$pp^{7m}G_{(2'\text{ and/or }3'OC(=O)R')}$ + $pN_{(2'OMe)}pN$ $\rightarrow$ $^{7m}G_{(2'\text{ and/or }3OC(=O)R')}pppN_{(2'OMe)}pN$

In Reaction 2 above, G is guanosine, $^{7m}G$ is 7-methylguanosine, N is any one of guanine, adenine, cytosine, and uracil, p is -P (=O)$O_2$-, R' is C1 to C6alkyl or C1 to C6alkoxy, and Im is an imidazolide. In the method of preparing the compound of Formula 1, the synthesis of intermediates $p^{7m}G_{(2'\text{ and/or }3'OC(=O)R')}$ and $pp^{7m}G_{(2'\text{ and/or }3'OC(=O)R')}$ may each be performed *In situ*.

A method of preparing intermediate $p^{7m}G_{(2'\text{ and/or }3'OC(=O)R')}$, which is a mono ester of intermediate $p^{7m}G_{(2'\text{ or }3'OC(=O)R')}$, wherein an embodiment includes a method of preparing a compound of Formula 6 (corresponding to $p^{7m}G_{(2'\text{ or }3'OC(=O)R')}$) or a salt thereof, including steps of

preparing a compound of formula 4 or a salt thereof by reacting a compound of Formula 2 below or a salt thereof with a compound of Formula 3 below under alkaline conditions in the aqueous phase; and

reacting the compound of Formula 4 or its salt with the compound of Formula 5 under conditions of pH 1.5 to 4.5 *In situ:*

[Formula 2]

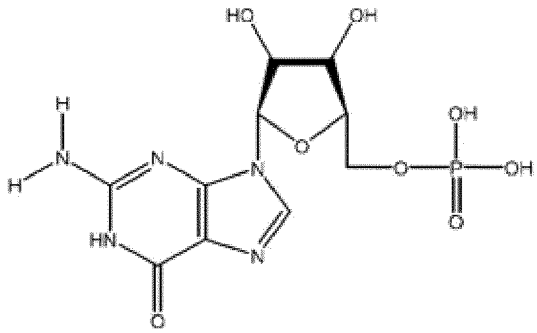

[Formula 3]

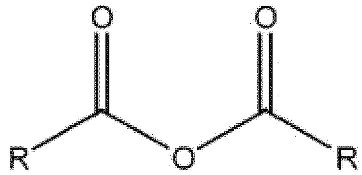

[Formula 4]

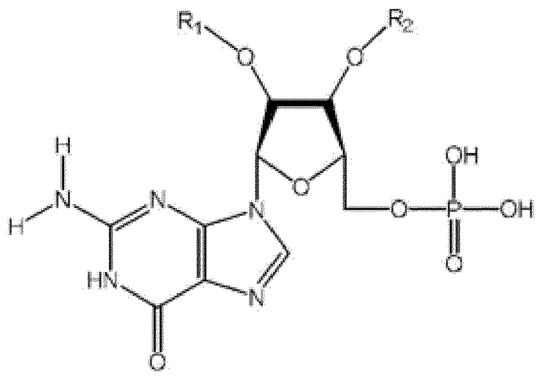

[Formula 5]

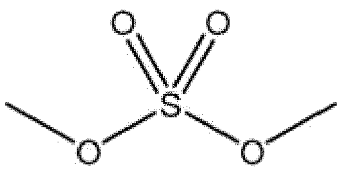

[Formula 6]

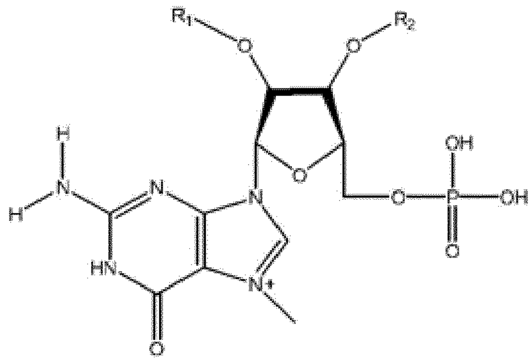

in the above formula, R refers to $C_1$ to $C_6$alkyl or $C_1$ to $C_6$alkoxy, and $R_1$ and $R_2$ are each independently H or C(=O)-R', wherein one of $R_1$ and $R_2$ is H and the other is C(=O)-R.

In an embodiment, the alkaline condition may specifically be pH 9.5 to 11.5, and may be formed, for example, using sodium hydroxide. In an embodiment, the pH of 1.5 to 4.5, which is a slightly acidic condition, may be formed, for example, using acetic acid.

A method of preparing intermediate $pp^{7m}G_{(2' \text{ and/or } 3'OC(=O)R')}$, which is a mono ester of intermediate $pp^{7m}G_{(2' \text{ or } 3'OC(=O)R')}$, wherein an embodiment includes a method of preparing a compound of Formula 8 (corresponding to $pp^{7m}G_{(2' \text{ or } 3'OC(=O)R')}$) or a salt thereof, including the steps of

preparing a compound of Formula 7 or a salt thereof by reacting a compound of Formula 6 or a salt thereof with imidazole; and

reacting the compound of formula 7 or a salt thereof with zinc chloride and triethylammonium phosphate *In situ:*

[Formula 7]

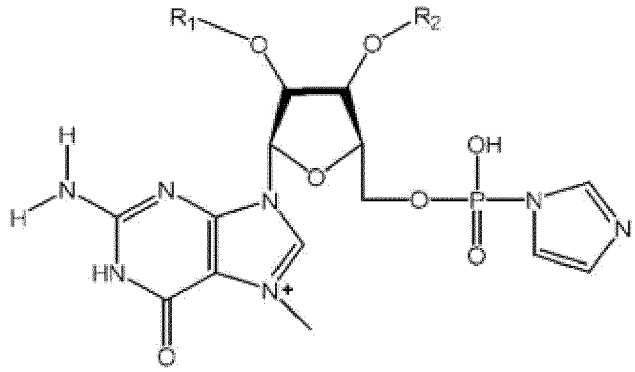

[Formula 8]

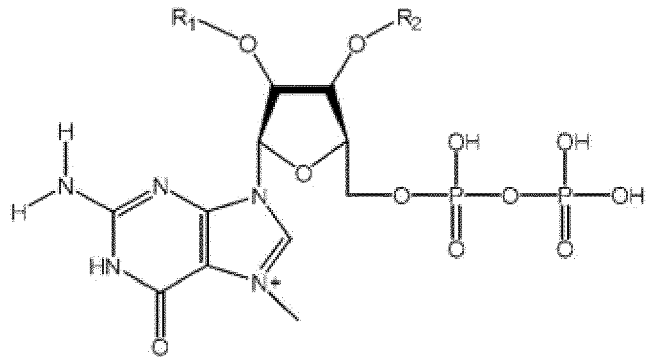

in the above formula, R refers to $C_1$ to $C_6$alkyl or $C_1$ to $C_6$alkoxy, and $R_1$ and $R_2$ are each independently H or C(=O)-R, wherein one of $R_1$ and $R_2$ is H and the other is C(=O)-R.

In the process of preparing a compound of formula 7 above or a salt thereof, triphenylphosphine, 2,2-dipyridyl disulfide, and triethylamine as an activation reagents to assist imidazole coupling may be reacted together with imidazole.

One embodiment of the compound of Formula 1 may be prepared according to the method shown in Reaction 3 below.

[Reaction 3]

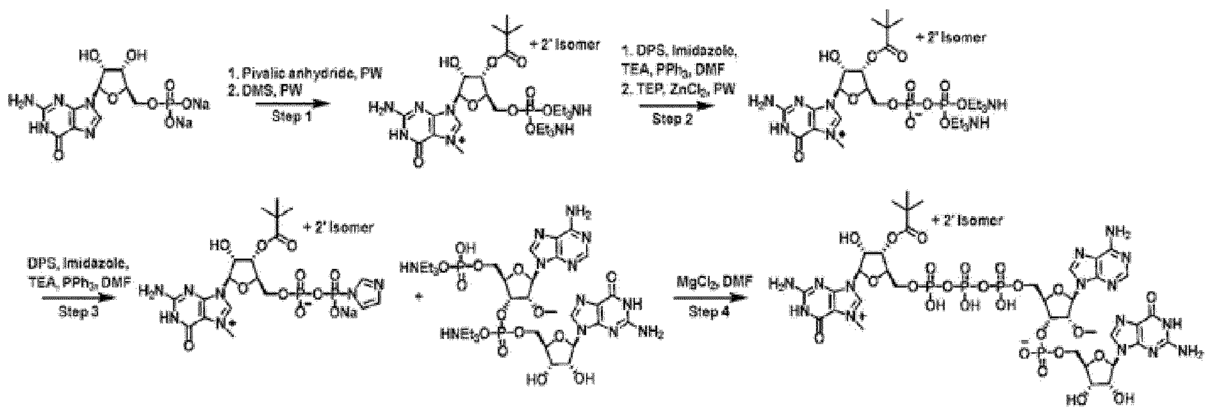


In Reaction 3, "2'isomer" refers to a 2' structural isomer in which the pivaloyl group at the 3' position is present at the 2' position instead of the 3' position, and "+2'isomer" refers to a mixture of 2' structural isomers existing together.

Details of a specific exemplified preparation method of the above Reaction 3 are described in the following embodiment.

Reaction 3 above may be briefly expressed with the following abbreviation.

[Reaction 3] pG → $p^{7m}G_{(2' \text{ or } 3'\text{monpivalic})}$ → $pp^{7m}G_{(2' \text{ or } 3'\text{monopivalic})}$ → Im-$pp^{7m}G_{(2' \text{ or } 3'\text{monopivalic})}$ + $pA_{(2'OMe)}pG$ → $^{7m}G_{(2' \text{ or } 3'\text{monopivalic})}pppA_{(2'OMe)}pG$

Wherein G refers to guanosine, 7mG refers to 7-methylguanosine, A refers to adenosine, p refers to -P(=O)(OH)O-, Me refers to methyl, and Im refers to an imidazolide.

The production method shown in Reaction 2 and Reaction 3 produces p7mG(2' and/or 3'OC(=O)R') by carrying out the monoesterification (or deesterification) and 7-methylation processes of guanosine *In situ,* the manufacturing process of 7-methylguanosine diphosphate (pp7mG(2' and/or 3'OC(=O)R')) may also be shortened by imidazolidation and diphosphorylation *In situ,* and the synthesis process up to 7-methylguanosine diphosphate (pp7mG(2' and/or 3'OC(=O)R')) may be shortened by two steps compared to the conventional method of preparing trinucleotide cap analogs of TriLink (the method of [Reaction 1-1] below).

That is, the compound of Formula 1 may be prepared in a total of four steps, and purification may be performed after each process, and then the next process may be performed. Therefore, four purification processes are required. This is a reduction of one purification process compared to TriLink's conventional commercialized cap analog production method, which requires five intermediate purification processes. Therefore, the compound of Formula 1 may be prepared more economically by reducing the number of processes and purification compared to conventionally known cap analog production methods.

The purification method may be performed independently at each steps through ion chromatography, reverse phase chromatography, etc. In an embodiment, the purification method is reverse phase chromatography.

In addition, in the case of a conventional second or third-generation cap analog, the largest portion of the net raw material cost for manufacturing cap analogs is the unit cost of the starting substance, 3'-O-methylguanosine nucleoside (G(3'OMe)), which requires a difficult process to selectively methylate only the 3'-hydroxyl of the three hydroxyl groups of guanosine nucleoside, and a purification process to selectively purify only the 3'-monoesterification product. On the other hand, the compound of Formula 1 may be prepared using inexpensive guanosine monophosphate as a starting substance. Specifically, the compounds of Formula 1 according to an embodiment that has a monoester form with only one -OC(=O)R' at the 2' or 3' position may be prepared by introducing a reaction that spontaneously substitute the hydroxy group at 2' or 3' of the guanosine monophosphate with -OC(=O)R' such that the reaction proceeds only to either 2' or 3'. In other words, there is no need to make efforts to selectively proceed with the reaction at 3'. Moreover, the compound of Formula 1, in the form of a monoester having only one -OC(=O)R' at the 2' or 3' position, maintains a mixture of structural isomers in which -OC(=O)R" is present at the 2' or 3' position, in dynamic equilibrium with each other. Therefore, there is no need to purify only one structural isomer. In other words, there is no need to go through a difficult process to selectively esterify only the hydroxyl at a specific position, and there is no need to go through a purification process to selectively purify only the specific monoesterification product. As a result, the desired compound of Formula 1 may be prepared using low-cost starting substances, thereby significantly reducing manufacturing costs.

In addition, mRNA capped with the monoester form of the compound of Formula 1 has a higher protein expression efficiency than when capped with the conventional trinucleotide 3'-methoxy cap analog of TriLink ($^{7m}G_{(3'OMe)}ppp A_{(2'OMe)}pG$), which ultimately reduces the cost required for protein synthesis.

In addition to the monoester form of the compound of Formula 1, the compound of Formula 1 in the form of a diester in which both 2'-OH and 3'-OH are esterified, which is another embodiment of the compound of Formula 1, since the target product is a diesterized product, there is an advantage that it may be prepared without monoesterification at the selective position and subsequent purification of the monoester.

Finally, considering that the price of 3'-O-methylguanosine nucleoside, which is the starting substance for the production of a conventional cap analog, is about 380 times higher per kg than guanosine monophosphate, which is the starting substance for the production of the cap analogs of the present disclosure, compounds of Formula 1 may be prepared with significantly higher economic efficiency compared to conventional cap analog compounds.

As described above, the cap analog, which is a compound of Formula 1, may be prepared economically in terms of time and cost by reducing the number of synthesis steps and purification compared to the conventional trinucleotide cap analog of TriLink. Additionally, the purification process may be performed using a reversed phase column, and since reversed phase column is more efficient than a conventional ion exchange column, the reversed phase column purification process may also further contribute to the economic efficiency of the preparation method of the compound of Formula 1. Additionally, a more detailed explanation is as follows.

In an embodiment of the method of preparing the compound of Formula 1, guanosine monophosphate sodium salt, which is an inexpensive and readily available raw material, is used as a starting substance, and the monoesterification and 7-methylation of guanosine is carried out *In situ* to obtain $p^{7m}G(_{2'\ or\ 3'monopivalic)}$, the process for preparing 7-methylguanosine diphosphate ($pp^{7m}G_{(2'\ or\ 3'monopivalic)}$) may also be shortened to an *In situ* imidazolidation and diphosphorylation, thus reducing the synthesis steps to 7-methylguanosine diphosphate ($pp^{7m}G_{(2'\ or\ 3'monopivalic)}$) by two processes compared to the conventional method of preparing a trinucleotide cap analog of TriLink (the method of [Reaction 1-1] below).

[Reaction 1-1] $G_{(3'OMe)} \rightarrow pG_{(3'OMe)} \rightarrow Im\text{-}pG_{(3'OMe)} \rightarrow ppG_{(3'OMe)} \rightarrow pp^{7m}G_{(3'OMe)} \rightarrow Im\text{-}pp^{7m}G_{(3'OMe)} + pN_{(2'OMe)}pN \rightarrow {}^{7m}G_{(3'OMe)}pppN_{(2'OMe)}pN$

In addition, the manufacturing method of TriLink's trinucleotide cap analog $^{7m}G_{(3'OMe)}pppN_{(2'OMe)}pN$ requires a total of 5 ion exchange column purifications in Reaction 1-1 (purification target: $pG_{(3'OMe)}$, $ppG_{(3'OMe)}$, $pp^{7m}G_{(3'Ome)}$, $pN_{(2'OMe)}pN$, $^{7m}G_{(3'OMe)}pppN_{(2'OMe)}pN$) (US 10,913,768 reference), while the compound of Formula 1 may be prepared through a total of 4 reversed-phase column purifications in Reaction 2 (purification target: $p^{7m}G_{(2'and/or\ 3'C(=O)O\text{-}R')}$, $pp^{7m}G_{(2'and/or\ 3'C(=O)O\text{-}R')}$, $pN_{(2'OMe)}pN$, $^{7m}G_{(2'and/or\ 3'C(=O)O\text{-}R')}pppN_{(2'OMe)}pN$). That is, the total number of purifications in the entire process may be reduced by one. In addition, while the conventional cap analog synthesis method requires the product at each process to undergo ion exchange column purification, the method for synthesizing the compound

of Formula 1 herein, the product in each process may be purified only using a reversed-phase column in which the injection amount of the purification target per column volume is about 10 times higher than that of an ion exchange column of the same volume. Therefore, the method of preparing the compound of Formula 1 may reduce the cost of raw materials for purification including triethylamine, used in the buffer, or resin, etc. by 60 % to 80 %. In the method of preparing the compound of Formula 1, the reaction is accomplished by substituting the highly polar 2' or 3' hydroxyl group of 7-methylguanosine with a highly non-polar alkyl ester group or alkoxy ester, as a result, the non-polarity of the product at each stage increased, making it possible to separate the product by non-polarity in a reversed-phase column instead of the conventional process in which the product at each process of manufacturing the cap analog had to be separated in an ion column according to the degree of ionization.

The compounds of Formula 1 may be used as a cap analog for 5' capping of mRNA during in vitro mRNA synthesis. That is, during in vitro mRNA synthesis, the compound of Formula 1 may be simultaneously introduced as a cap analog to synthesize an mRNA whose 5' end consists of a cap analog.

Another aspect provides a cap analog including the compound of Formula 1 above.

Specifically, the cap analog may be the compound of Formula 1 itself, or, in addition to the compound of Formula 1, may additionally include a hybridization sequence that may be complementary to the sequence on the DNA template at the initiation site. The length of the hybridization sequence of a cap analog for use in the methods and compositions provided herein depends on several factors, including the identity of the template nucleotide sequence and the temperature at which such primers are hybridized to the DNA template or used during in vitro transcription. The desired length of the hybridized nucleotide sequence of the cap analog for use in transcription may be readily determined by conventional experiments by one of ordinary skill in the art. For example, the length of hybridizing nucleotides may be determined based on the desired hybridization specificity or selectivity.

In an embodiment, the cap analog has a nucleotide length (including the cap) of about 3 to about 9 nucleotides, more specifically about 3 to about 7 nucleotides, and even more specifically about 3 to about 5 nucleotides. In an embodiment, the cap analog has a nucleotide length of 3 nucleotides. The length of the hybridization sequence within the cap analog may be equal to or shorter than the overall length of the cap analog.

Using the cap analogs, 5' capped mRNA may be prepared.

Accordingly, one particular aspect provides a method of preparing 5' capped mRNA including incorporating a cap analog including a compound of Formula 1 above during synthesis of the mRNA.

Additionally, one particular aspect provides a composition or kit for preparing a 5' capped mRNA, including a cap analog including the compound of Formula 1 above.

Additionally, one particular aspect provides the use of a cap analog including a compound of Formula 1 for use in the synthesis of mRNA.

Additionally, one particular aspect provides an mRNA 5'-capped with a cap analog including a compound of Formula 1 above.

The method of preparing a 5' capped mRNA using a cap analog including the compound of Formula 1 may be performed according to any method known in the art.

In an embodiment, the 5' capped mRNA may be prepared by a co-transcriptional capping method, which is a method of mRNA synthesis in which a chemically synthesized cap analog is simultaneously introduced during in vitro mRNA synthesis and the cap analog constitutes the 5' end. Specifically, the method may include steps of introducing a cap analog that is a compound of Formula 1 into a mixture including an RNA polymerase under conditions in which transcription of a polynucleotide template occurs by the RNA polymerase; and incubating the mixture for a time sufficient to allow transcription of the template.

A cap analog including a compound of Formula 1 according to an aspect may increase the efficiency of transcription of an in vitro mRNA compared to the efficacy of initiation utilizing standard GTP, ATP, CTP or UTP, and subsequently increase the efficiency of protein expression of the transcribed capped mRNA during translation. Due to the increase in transcriptional efficiency, the synthesis of the mRNA may be increased by, for example, about 10 %, about 20 %, about 40 %, about 60 %, about 80 %, about 90 %, about 100 %, about 150 %, about 200 %, or about 500 % compared to synthesizing the mRNA by typical methods.

A cap analog including a compound of Formula 1 according to an aspect may increase protein expression of a 5' capped mRNA molecule to a level higher than a conventional trinucleotide 3'-methoxy cap analog of TriLink ($^{7m}G_{(3'OMe)}pppA_{(2'OMe)}pG$). More specifically, a cap analog monoesterified with only one of the 2' or 3' OH residues of the compound of Formula 1 may increase protein expression of a 5' capped mRNA molecule to a significantly higher level than the conventional TriLink trinucleotide 3'-methoxy cap analog ($^{7m}G_{(3'OMe)}pppA_{(2'OMe)}pG$) (see Experimental Example 2).

Therefore, a cap analog including a compound of Formula 1 according to an aspect has a protein expression efficiency equal to or greater than that of a third-generation cap analog, and may have the advantage of reducing the number of manufacturing processes and purification processes of the cap analog itself, and increasing the purification efficiency of the cap analog itself, thereby lowering the manufacturing cost.

In an embodiment, a method of preparing a 5' capped mRNA may further include adding a at least one modified NTP to the transcription reaction. The modification of the at least one modified NTP does not substantially impair RNA polymerase-mediated synthesis of the mRNA. The modified NTP may include, for example, one or more modified nucleoside bases, one or more modified sugars, or one or more modified 5'-triphosphates. This modified NTP may be incorporated onto the 3'-end of the cap analog, which supports additional elongation of a primer without blocking transcription. The modifying group of the modified NTP may be a detectable label or a detectable marker. Therefore, after transcription, the produced mRNA containing a detectable label or marker may be identified by size, mass, color, and/or affinity capture. In an embodiment, the detectable label or marker is a fluorescent dye; and the affinity capture label is biotin.

In an embodiment, one or more components of the transcription reaction (cap analog and/or NTP) may be labeled with a detectable label or marker. Therefore, after transcription, the mRNA molecule may be identified, for example, by size, mass, affinity capture, or color. For example, the detectable label is a fluorescent dye; the affinity capture label is biotin.

The kit or composition for preparing 5' capped mRNA may contain all transcription reagent for the synthesis of a typical mRNA (for example, FLuc mRNA). More specifically, the kit may include a cap analog; a container labeled for transcription; instructions for performing the mRNA synthesis; and one or more reagents selected from the group consisting of one or more unmodified NTPs, one or more modified NTPs (for example, methylpseudouridine 5'-triphosphate), RNA polymerase, other enzymes, reaction buffer, magnesium, and DNA template.

A 5' capped mRNA prepared using a cap analog according to an aspect, including the cap analog within its structure, may be used to administer the 5' capped mRNA to an organism to express a protein in vivo.

Thus, another aspect provides a method of expressing a peptide or protein of interest in vivo by administering the mRNA 5'-capped with the cap analog in vivo.

Additionally, another aspect provides a pharmaceutical composition for expressing a peptide or protein of interest, including an mRNA 5'-capped with the cap analog and a pharmaceutically acceptable carrier.

Depending on the type of peptide or protein of interest, the desired disease treatment or prevention effect may be obtained in vivo. Therefore, it may be used for the treatment or prevention of any disease that may be treated or prevented by expression of the peptide or protein. Diseases that may be treated or prevented by expression of a specific type of peptide or protein are known, and the pharmaceutical composition may be used to prevent or treat the target disease by inducing the expression of the peptide or protein.

Additionally, another aspect provides the medicinal use of an mRNA 5'-capped with the cap analog for the prevention or treatment of any disease effected by in vivo expression of the peptide or protein.

The pharmaceutical composition and treatment or prevention method may be used for gene replacement therapy, genome editing, cancer immunotherapy, or treatment or prevention, and the like, using a vaccine. In an embodiment, the pharmaceutical composition is an mRNA vaccine.

The pharmaceutical composition may be formulated for administration by injection or other appropriate route known to person of ordinary skill in the art to treat or prevent a particular condition. Compositions for injection include a pharmaceutically acceptable carrier, for example a sterile saline solution. Composition for injection may also be formulated as a suspending agent in a lipid or phospholipid, a liposomal suspension, or an aqueous emulsion. Methods of formulating the pharmaceutical composition are widely known to those of ordinary skill in the art.

In an embodiment, the pharmaceutical composition may contain mRNA including a cap analog as the active ingredient at a concentration of about 0.01 % to 1 %. The concentration may vary depending on frequency of administration, administration dosage, method of administration, and the like.

In an embodiment, the pharmaceutical composition may be administered to a mammal, more specifically a human, wherein the dosage may vary depending on the health status of the individual, the severity of the disease, body weight, age, race, and the like, and an appropriate dosage may be determined by a person skilled in the art. In an embodiment, the dosage for humans is in the range of 0.0001 to 100 mg/day, and more specifically in the range of about 0.1 to 50 mg/day.

The mRNA 5'-capped with a cap analog according to an aspect may be introduced into a cell to express a protein or peptide in vivo or in vitro.

Thus, another aspect provides a cell containing an mRNA 5'-capped with a cap analog according to an aspect.

Another aspect provides a cell containing a protein or peptide translated from an mRNA 5'-capped with a cap analog according to the aspect above.

Methods for expressing protein peptides in cells in vivo or in vitro using the mRNA are known in the art, and proteins or peptides may be appropriately expressed in cells according to such conventional typical methods.

[Example]

Hereinafter, embodiments are presented to facilitate understanding of the present disclosure. The following examples are merely illustrative of the present invention, and it is clear to those skilled in the art that various changes

and modifications are possible within the scope and spirit of the present invention, and it is natural that such changes and modifications fall within the scope of the appended patent claims.

## Explanation of Abbreviations

The following abbreviations are used herein.

TBSCl: Tris-butyl dimethyl silyl chloride
DMAP: 4-Dimethylaminopyridine
DMSO: Dimethyl sulfoxide
TFA: Trifluoroacetic acid
DCM: Dichloromethane
TEP: Triethyl phosphate
DPS: 2,2'-Dipyridyl disulfide
$PPh_3$: Triphenylphosphine
DMF: Dimethylformamide
DMS: Dimethyl sulfate
PW: Pure water
EDTA: Ethylenediaminetetraacetic acid
THF: Tetrahydrofuran
ACN: Acetonitrile
TEA: Triethylamine
TCA: Trichloroacetic acid
IVT: In vitro transcription

**Example** 1 to **Example** 7: **Preparation** of **7-methyl-2',3'-pivaloyl-guanosine 5'-diphosphate imidazolide (im-pp$^{7m}$G(2',3'Pivaloyl)) (7)**

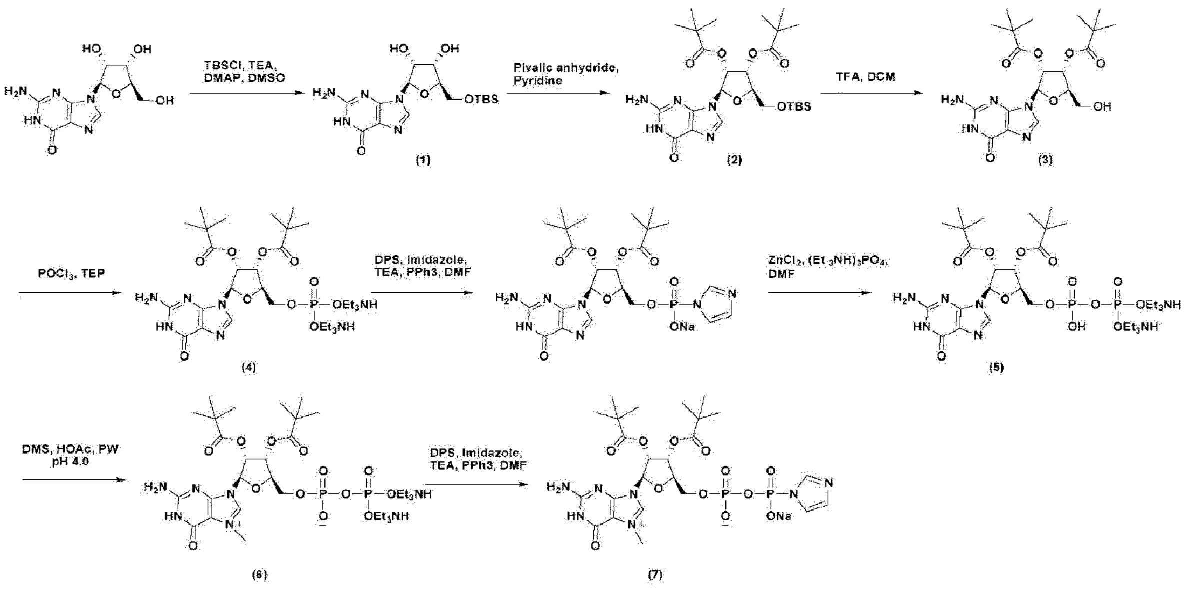


**Example** 1: **Preparation of 5'-O-tert-butyldimethylsilyl-guanosine (1)**

After dissolving 20 g (70.6 mmol) of guanosine in 200 ml of dimethyl sulfoxide, 21.6 g (155.4 mmol) of triethylamine, 21.2 g (141.2 mmol) of tert-butyldimethylsilyl chloride, and 860 mg (7 mmol) of dimethylaminopyridine were added under nitrogen. After stirring at room temperature for 24 hours, it was slowly added dropwise to distilled water at 0 to 5 °C, and the generated precipitate was filtered. The filtered precipitate was purified using flash column chromatography to obtain 24.4 g (87 %) of the target compound (1).

$^1$H NMR (400 MHz, $CD_3OD$, 25°C): $\delta$= 7.96 (s, 1H), 5.89 (d, 1H), 4.47 (t, 1H), 4.33 (t, 1H), 4.08 (m, 1H), 3.94

~ 3.87 (m, 2H), 0.94 (m, 9H), 0.12 (m, 6H).

**Example 2: Preparation** of **2',3'-pivaloyl-5'-O-tert-butyldimethylsilyl-guanosine (2)**

10 g (25.2 mmol) of 5'-O-tert-butyldimethylsilyl-guanosine (1) was dissolved in 100 ml of anhydrous pyridine, and 23.4 g (125.6 mmol) of pivalic anhydride was added and stirred at room temperature. After completion of the reaction, 500 ml of ethyl acetate was added, washed with 400 ml of saturated sodium bicarbonate aqueous solution and 400 ml of 20 % citric acid, dried with magnesium sulfate, and distilled under reduced pressure. Flash column chromatography was used to obtain 10.26 g (72 %) of the target compound (2).

$^1H$ NMR (400 MHz, DMSO-d6, 25°C): $\delta$= 10.70 (s, 1H), 7.83 (d, 1H), 6.51 (br, 2H), 5.93 (d, 1H), 5.69 (m, 1H), 5.40 (m, 1H), 4.21 (m, 1H), 3.86 (m, 2H), 1.20 (s, 9H), 1.04 (s, 9H), 0.88 (s, 9H), 0.08 (s, 6H).

**Example 3: Preparation of 2',3'-pivaloyl-guanosine (3)**

10 g (17.6 mmol) of 2',3'-pivaloyl-5'-O-tert-butyldimethylsilyl-guanosine (2) was dissolved in 300 mL of dichloromethane, and 8 ml of distilled water and 70 ml of trifluoroacetic acid were sequentially added. After stirring for 30 minutes, it was neutralized with 2N sodium hydroxide. The organic layer and the aqueous layer were separated, the aqueous layer was extracted with 300 ml of dichloromethane, and the organic layer was collected, washed with saturated sodium bicarbonate aqueous solution and brine, dried with magnesium sulfate, and distilled under reduced pressure. Flash column chromatography was used to obtain 5.16 g (64.7 %) of the target compound (3).

$^1H$ NMR (400 MHz, DMSO-d6, 25°C): $\delta$= 10.69 (s, 1H), 7.97 (d, 1H), 6.49 (br, 2H), 5.92 (d, 1H), 5.72 (dd, 1H), 5.45 (t, 1H), 5.40 (dd, 1H), 4.16 (m, 1H), 3.67 (m, 2H), 1.21 (m, 9H), 1.05 (m, 9H).

**Example 4: Preparation of 2',3'-pivaloyl-guanosine 5'-monophosphate (pG(2',3' Pivaloyl)) (4)**

4.51 g (10 mmol) of 2',3'-pivaloyl-guanosine (3) was added to 40 mL of triethyl phosphate and dissolved at 60 to 70 °C. The reaction solution was cooled to 0 °C, phosphorus oxychloride 2.8 mL (30 mmol) was added, and then warmed to room temperature and stirred under nitrogen for 3 hours. 100 mL of 1M triethylammonium bicarbonate buffer (pH 8.5) was gradually added dropwise, stirred for 8 hours, and then 1 L of distilled water was added. The reaction solution was separated using a C18 column (50 x 250 mm) and azeotroped with 50 mL of methanol four times to give 3.79 g (60.0 % yield) of the triethylammonium salt (4) of the target compound.

$^1H$ NMR (400 MHz, DMSO-d6, 25°C): $\delta$= 10.72 (s, 1H), 8.03 (s, 1H), 6.68 (br, 2H), 5.94 (d, 1H), 5.83 (m, 1H), 5.46 (m, 1H), 4.28 (m, 1H), 4.00 (m, 2H), 1.21 (m, TEA overlapped, 9H), 1.03 (m, 9H). LC-MS (ESI, m/z) = 532.17 [M + $H^+$]

**Example 5: Preparation** of **2',3'-pivaloyl-guanosine 5'-diphosphate (ppG(2',3' Pivaloyl)) (5)**

To 250 mL of dimethylformamide, 3.16 g (5.0 mmol) of pG(2',3' Pivaloyl) triethylammonium salt (4) was added, followed by adding imidazole 3.40 g (50 mmol), triphenylphosphine 6.56 g (25 mmol), 2,2'-dipyridyl disulfide 5.51 g (25 mmol), and triethylamine 0.51 g (5.0 mmol) was stirred for 5 hours. The reaction solution was added to a solution of 2.45 g (20 mmol) of sodium perchlorate dissolved in 430 mL of acetone, cooled to 4°C, and the generated crystal was filtered, washed with cold acetone, and vacuum dried. The dried im-pG (2',3' Pivaloyl) was added to 30 mL of dimethylformamide, 1.36 g (10 mmol) of zinc chloride and 5.98 g (30 mmol) of triethylammonium phosphate were added, and then stirred at room temperature for 3 to 16 hours. After completion of the reaction, a mixture of 870 mL distilled water, 9.79 g (33.5 mmol) and ethylenediaminetetraacetic acid was added to the reaction solution and stirred for 20 minutes. The reaction solution was neutralized to pH 6 to 7 using 1 M sodium bicarbonate aqueous solution, and then the reaction solution was separated using a C18 column (50 $\times$ 250 mm) and azeotroped with 35 mL of methanol four times to give 2.44 g (60.0 % yield) of the triethylammonium salt (5) of the target compound.

$^1H$ NMR (400 MHz, $D_2O$, 25°C): $\delta$= 7.99 (s, 1H), 6.00 (d, 1H), 5.71 (m, 1H), 5.52 (m, 1H), 4.49 (m, 1H), 4.16 (m, 2H), 1.11 (m, TEA overlapped, 9H), 0.95 (m, 9H). $^{31}P$ NMR (162 MHz, $D_2O$, 25°C): $\delta$= -8.38 (d, 1P), -10.69 (d, 1P).

**Example 6: Preparation of 7-methyl-2',3'-pivaloyl-guanosine 5'-diphosphate (pp$^{7m}$G(2',3' Pivaloyl)) (6)**

2.44 g (3.0 mmol) of ppG(2',3' Pivaloyl) triethylammonium salt (5) was dissolved in 50 mL of distilled water and the pH of the reaction solution was adjusted to 4.0 using glacial acetic acid. To the reaction solution, 10 mL (105.45 mmol) of dimethyl sulfate was added gradually over 30 minutes and stirred for 4 hours. At this time, the pH of the reaction solution was maintained at 4.0 $\pm$ 0.5 using 0.1 N sodium hydroxide. The reaction solution was extracted three times with 150 mL of dichloromethane to remove unreacted dimethyl sulfate, and the aqueous layer was adjusted to pH 5.5 and 500 mL of distilled water was added. The reaction solution was separated using a C18 column (50 x 250 mm) to

obtain $pp^{7m}G$(2',3' Pivaloyl), which was distilled and vacuum dried to obtain 1.74 g (80 % yield) of the triethylammonium salt (6).

$^{1}H$ NMR (400 MHz, $D_2O$, 25°C): δ= 9.35 (s, 1H), 6.30 (d, 1H), 5.71 (m, 1H), 5.58 (m, 1H), 4.69 (m, 1H), 4.32 ~ 4.15 (m, 2H), 4.11 (s, 3H), 1.25(m, 9H), 1.19 (m, 9H). LC-MS (ESI, m/z) = 624.14 $[M - H]^-$

**Example 7: Preparation of 7-methyl-2',3'-pivaloyl-guanosine 5'-diphosphate imidazolide (im-$pp^{7m}$G(2',3'Pivaloyl)) (7)**

1.26 g (1.73 mmol) of $pp^{7m}G$(2',3' Pivaloyl) triethylammonium salt (6) was dissolved in 25 mL of dimethylformamide, followed by adding imidazole 941.63 mg (13. 83 mmol), 2,2'-dipyridyl disulfide 1.14 g (5.19 mmol), triethylamine 0.482 mL (3.46 mmol), and triphenylphosphine 1.36 g (5.19 mmol) was stirred for 6 to 8 hours. The reaction solution was added to a solution of 847 mg (6.92 mmol) of sodium perchlorate dissolved in 175 mL of acetone, cooled to 4 °C, and the generated crystal was filtered off, washed with cold acetone, and vacuum dried to obtain 967 mg (80.0 % yield) of the target compound (7).

**Example 8 to Example 10: Preparation of 7-methyl-3'-pivaloyl-guanosine 5'-diphosphate imidazolide (im-$pp^{7m}$G(2' or 3'monopivaloyl)) (10)**

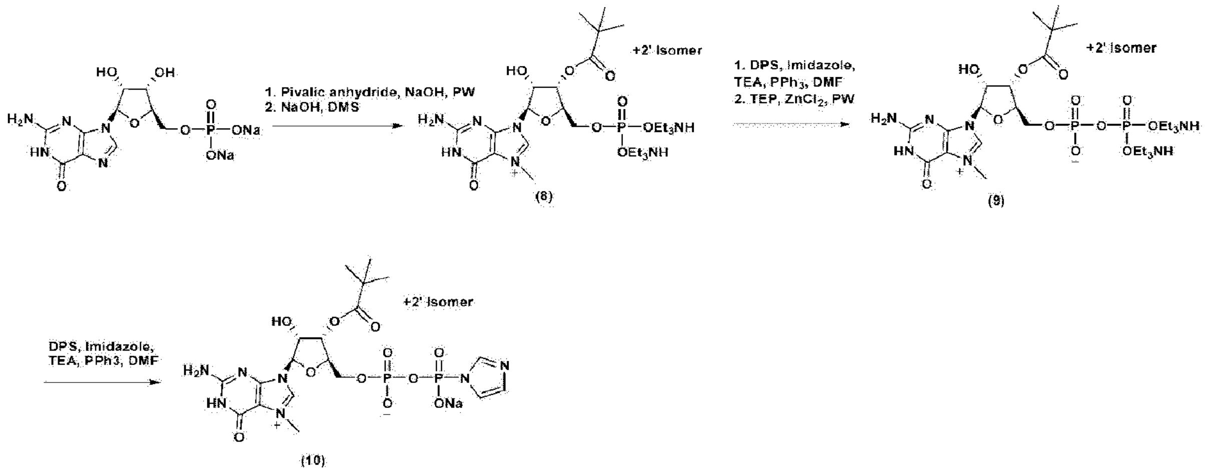


(8) (9) (10)

**Example 8: Preparation of 7-methyl-2' or 3'-monopivaloyl-guanosine monophosphate ($p^{7m}$G(2' or 3'monopivaloyl))** (8)

Commercially available guanosine monophosphate was used as a starting substance without additional treatment. 5 g (11.8 mmol) of guanosine monophosphate was dissolved in 50 ml of distilled water and titrated to pH 9.5 using 1 M sodium hydroxide. 8.79 g (47.2 mmol) of pivalic anhydride was added at ambient temperature and stirred at room temperature. The reaction was performed at ambient temperature using 1M sodium hydroxide, maintaining pH 9.5 to 9.6 for 4 to 12 hours. After completion of the reaction, 50 % acetic acid was added to titrate the pH to 4.5, and then 14.9 g (118 mmol) of dimethyl sulfate was gradually added over 30 minutes and stirred for 4 hours. At this time, the pH of the reaction solution was maintained at 4.0 ± 0.5 using 1 M sodium hydroxide. The reaction solution was extracted three times with 150 mL of dichloromethane to remove unreacted dimethyl sulfate, and the aqueous layer was titrated to pH 5.5. The reaction solution was separated using a C18 column (50 x 250 mm) to give the title reaction product (a mixture of 2'-monopivaloyl compound:3'-monopivaloyl compound = 0.6±0.05:1 by molar ratio (FIG. 1)), which was distilled, vacuum dried to obtain 3.4 g (43 % yield) of the triethylammonium salt (8).

$^{1}H$ NMR (400 MHz, $D_2O$, 25°C): δ= 6.17 (m, 0.6H, from 2'-monopivaloyl compound), 6.08 (m, 1H, from 3'-monopivaloyl compound), 5.50 (m, 0.6H, 2'H from 2'-monopivaloyl compound), 5.33 (m, 1H, 3'H from 3'-monopivaloyl compound), 4.89 (m, 1H, from 3'-monopivaloyl compound), 4.65 (m, 0.6H, from 2'-monopivaloyl compound), 4.51 (m, 1H, from 3'-monopivaloyl compound), 4.39 (m, 0.6H, from 2'-monopivaloyl compound), 3.96 ~ 4.15 (m, 8H, 2'-monopivaloyl and 3'-monopivaloyl compounds, overlapped), 3.13 (q, 9.6H, 2'-monopivaloyl and 3'-monopivaloyl compounds, overlapped), 1.18 ~ 1.24 (m, 28.8H, 2'-monopivaloyl and 3'-monopivaloyl compounds, overlapped). $^{31}P$ NMR (162 MHz, $D_2O$, 25°C): δ= 3.75 ~ 3.83 (s, 1.6 P, 2'-monopivaloyl and 3'-monopivaloyl compounds, overlapped).

**Example 9: Preparation of 7-methyl-2'or 3'-monopivaloyl-guanosine 5'-diphosphate (pp$^{7m}$G(2'or 3' monopivaloyl)) (9)**

3.4 g (5.07 mmol) of p$^{7m}$G$_{(2'}$ or 3' monopivaloyl) triethylammonium salt (8) was added to 75 mL of dimethylformamide, followed by adding imidazole 2.07 g (30.4 mmol), triphenylphosphine 3. 99 g (15.2 mmol), 2,2'-dipyridyl disulfide 3.35 g (15.2 mmol) and triethylamine 0.51 g (5.07 mmol) and being stirred for 1 to 12 hours. Afterwards, 2.07 g (15.2 mmol) of zinc chloride and 6.06 g (30.4 mmol) of triethylammonium phosphate were added and stirred at room temperature for 3 to 16 hours. After completion of the reaction, a mixture of 0.94 L distilled water 9.93g (34 mmol) and ethylenediaminetetraacetic acid was added to the reaction solution and stirred for 20 minutes. The reaction solution was neutralized to pH 6 to 7 using 1 M sodium bicarbonate aqueous solution, and then the reaction solution was separated using a C18 column (50 × 250 mm) and azeotroped with 35 mL of methanol four times to give 3.02 g (80 % yield) of the triethylammonium salt (9) of the title reaction product (a mixture of 2'-monopivaloyl compound:3'-monopivaloyl compound in molar ratio = 0.7±0.05:1 (FIG. 2)).

$^1$H NMR (400 MHz, $D_2O$, 25°C): δ= 6.18 (m, 0.7H, 2'H from 2'-monopivaloyl compound), 6.10 (m, 1H, from 3'-monopivaloyl compound), 5.57 (m, 0.7H, 2'H from 2'-monopivaloyl compound), 5.37 (m, 1H, 2'H from 3'-monopivaloyl compound), 4.94 (m, 1H, from 3'-monopivaloyl compound), 4.72 (m, 0.7H, from 2'-monopivaloyl compound), 4.57 (m, 1H, from 3'-monopivaloyl compound), 4.41 (m, 0.7H, from 2'-monopivaloyl compound), 4.14 ~ 4.37 (m, 3.4H, 2'-monopivaloyl and 3'-monopivaloyl compounds, overlapped), 4.10 (m, 5.1H, 2'-monopivaloyl and 3'-monopivaloyl compounds, overlapped), 3.13 ~ 3.19 (m, 15.3H, 2'-monopivaloyl and 3'-monopivaloyl compounds, overlapped), 1.21 ~ 1.26 (m, 38.3H, 2'-monopivaloyl and 3'-monopivaloyl compounds, overlapped). $^{31}$P NMR (162 MHz, $D_2O$, 25°C): δ= -8.71 - -9.08 (m, 1.7P, 2'-monopivaloyl and 3'-monopivaloyl compounds, overlapped), -10.42 ~ -10.63 (m, 1.7P, 2'-monopivaloyl and 3'-monopivaloyl compounds, overlapped).

**Example 10: Preparation of 7-methyl-3'-pivaloyl-guanosine 5'-diphosphate imidazolide (im-pp$^{7m}$G(2' or 3'monopivaloyl)) (10)**

1.29 g (1.73 mmol) of pp$^{7m}$G$_{(2'}$ or 3' monopivaloyl) triethylammonium salt (9) was dissolved in 22 mL of dimethylformamide, followed by adding 707 mg (10.38 mmol) of imidazole and 1.14 g (5.19 mmol) of 2,2'-dipyridyl disulfide. 175 mg (1.73 mmol) of triethylamine and 1.36 g (5.19 mmol) of triphenylphosphine were added to the reaction solution and stirred for 1 to 12 hours. After completion of the reaction, the reaction solution was added to a solution of 847 mg (6.92 mmol) of sodium perchlorate dissolved in 175 mL of acetone, cooled to 4 °C, and the generated crystal was filtered off, washed with cold acetone, and vacuum dried to give 796 mg (75.0 % yield) of the target compound (10).

**Example 11 to Example 14: Preparation of pA$_{(2'OMe)}$pG (14)**

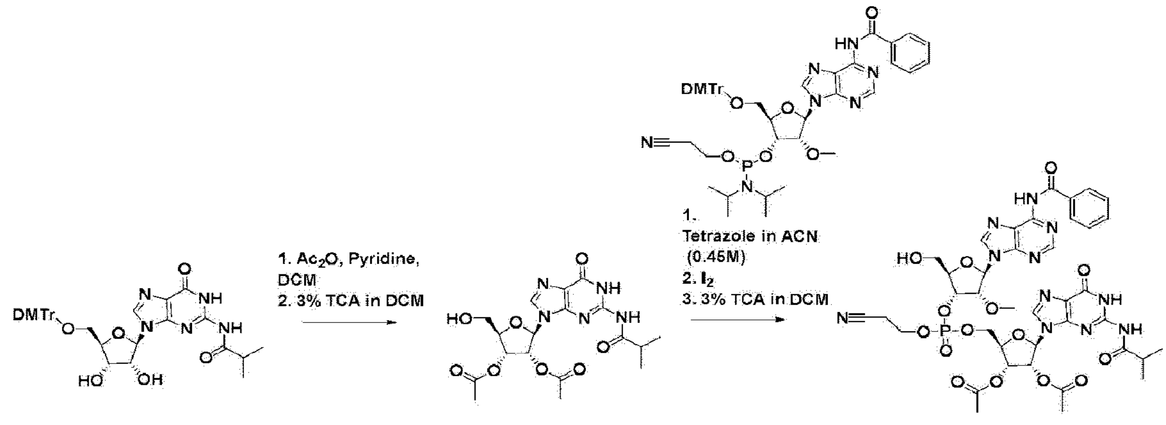


(11) (12)

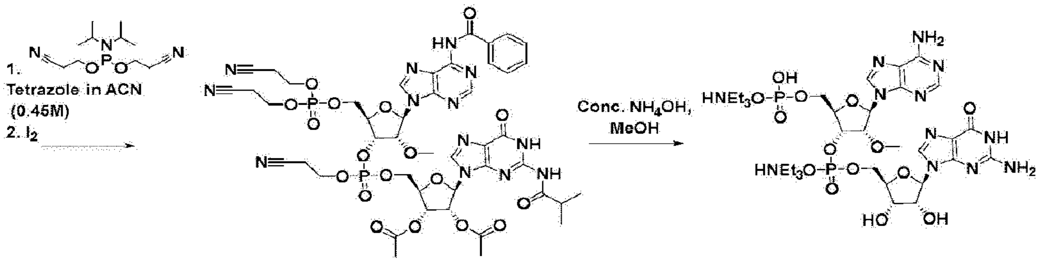


(13) (14)

**Example 11: Preparation of N2-isobutyryl-2',3'-diacetoxy-guanosine (11)**

N2-Isobutyryl-5'-O-DMT-guanosine 2.4 g (3.66 mmol) was dissolved in 12 ml of dichloromethane, followed by adding 1.47 ml (18.3 mmol) of pyridine, 1.78 ml (18.3 mmol) of anhydrous acetic acid and stirred at room temperature for 5 hours. 24 ml of ethyl acetate was added to the reaction solution, followed by extraction and washed with 14.4 ml of sodium bicarbonate saturated aqueous solution, 14.4 ml of 20 % citric acid aqueous solution, and 14.4 ml of distilled water. The organic layer was dried with sodium sulfate and the residue was distilled under reduced pressure and dried with nitrogen for 1 day. 36 ml of 3 % trichloroacetic acid aqueous solution was added to the dried material and reacted at room temperature for 3 hours, then 24 ml of methanol was added and stirred for an additional 2 hours and 30 minutes. The reaction solution was distilled under reduced pressure followed by adding 36 ml of dichloromethane and 18 ml of distilled water, and then neutralized by adding a sodium bicarbonate saturated aqueous solution. The organic layer was separated, dried with sodium sulfate and distilled under reduced pressure. The residue distilled under reduced pressure was dissolved in 12 ml of ethyl acetate and then gradually added to 108 ml of hexene at room temperature to crystallize. The generated crystal was filtered, washed three times with 12 ml of hexene and vacuum dried to obtain 1.43 g (89.6 % yield) of the target compound (11). LC-MS (ESI, m/z) = 438.16 [M + H$^+$]

**Example 12: Preparation of (N2-isobutyryl-2',3'-diacetoxy-guanosinyl)-N6-benzoyl-2'-methoxy-adenosinyl cyanoethyl phosphate ester (12)**

5'-O-DMT-N6-benzoyl-2'-methoxy-adenosine amidite was obtained commercially and used as a starting substance without additional treatment. N2-Isobutyryl-2',3'-diacetoxy-guanosine (11) 1.43 g (3.28 mmol) and 5'-O-DMT-N6-benzoyl-2'-methoxy-adenosine amidite 3.78 g (4.26 mmol) were dissolved in 28.4 mL of 1H-tetrazole (0.45 M acetonitrile solution, 12.79 mmol) and stirred at room temperature for 1 hour. Iodine 544 mg (2.15 mmol) was dissolved in 56.7 ml of tetrahydrofuran:distilled water:pyridine mixture (v:v:v, 7:2:1) and the solution was added to the reaction solution and stirred for 45 minutes. 7.2 ml of a 10 % sodium thiosulfate aqueous solution was added to the reaction solution, followed by adding 43 ml of dichloromethane and 14.3 ml of distilled water, and the organic layer was separated. The separated organic layer was dried with sodium sulfate, distilled under reduced pressure, and then dried with nitrogen for 1 day. 21.5 ml of 3 % trichloroacetic acid aqueous solution was added to the dried material and reacted at room temperature for 3 hours, then 14.3 ml of methanol was added and stirred for an additional 2 hours and 30 minutes. The reaction solution was distilled under reduced pressure followed by adding 21.5 ml of dichloromethane, 10.7 ml of distilled water, and then neutralized by adding a sodium bicarbonate saturated aqueous solution. The separated organic layer

was washed with distilled water, dried with sodium sulfate, and distilled under reduced pressure. The residue from the reduced pressure distillation was dissolved in 28.6 ml of dichloromethane and gradually added to 86 ml of methyl tertiary butyl ether at room temperature to crystallize. The generated crystal was filtered and vacuum dried for one day to obtain 2.7 g (87.8 % yield) of the target compound (12). LC-MS (ESI, m/z) = 938.28 [M + H$^+$]

**Example 13: Preparation of p(OCE)$_2$A$^{bz}$(2'OMe)p(OCE)G$^{ib}$(2',3'OAc) (13)**

(N2-Isobutyryl-2',3'-diacetoxy-guanosinyl)-N6-benzoyl-2'-methoxy-adenosinyl cyanoethyl phosphate ester (12) 2 g (2.13 mmol), and bis(2-cyanoethyl)-N,N-diisopropylphosphoamidite 1. 11 ml (4.26 mmol) were dissolved in 9.47 mL of 1H-tetrazole (0.45 M acetonitrile solution, 4.26 mmol) and stirred at room temperature for 30 minutes. Iodine 811 mg (3.2 mmol) was dissolved in 30 ml of tetrahydrofuran:distilled water:pyridine mixture (v:v:v, 7:2:1) and the solution was added to the reaction solution and stirred for 30 minutes. 20 ml of a 10 % sodium thiosulfate aqueous solution was added to the reaction solution, followed by adding 100 ml of dichloromethane and 40 ml of distilled water, and the organic layer was separated. The separated organic layer was dried with sodium sulfate and distilled under reduced pressure. The concentrate distilled under reduced pressure was dissolved by adding 27 ml of dichloromethane and then gradually added to 133 ml of methyl tertiary butyl ether at room temperature to crystallize. The generated crystal was filtered and vacuum dried for 1 day to obtain the target compound (N2-isobutyryl-2',3'-diacetoxy-guanosinyl)-N6-benzoyl-2'-methoxy-5'-dicyanoethylphosphoryl- adenosinyl cyanoethyl phosphate ester (13) 2.2 g (92.2 % yield). LC-MS (ESI, m/z) = 1124.30 [M + H$^+$]

**Example 14: Preparation of pA$_{(2'OMe)}$pG (14)**

2 g (1.78 mmol) of p(OCE)$_2$A$^{bz}$(2'OMe)p(OCE)G$^{ib}$(2',3'OAc) (13) was added to a mixture of 44 mL of methanol and 44 mL of concentrated ammonia and stirred at 50 to 55 °C for 24 hours. When the reaction was completed, the solvent was distilled under reduced pressure, and 20 mL of methanol was added to the concentrate and distilled three times. The concentrate was again dissolved in distilled water and purified using a DEAE Sepharose column and reverse phase chromatography to obtain1.13 g (70 % yield) of the triethylammonium salt (14) of the target compound.

$^1$H NMR (400 MHz, D$_2$O, 25°C): δ= 8.44 (s, 1H), 8.12 (s, 1H), 7.90 (s, 1H), 6.07 (m, 1H), 5.80 (m, 1H), 4.46 ~ 4.01 (m, 8H), 3.45 (m, 3H) LC-MS (ESI, m/z) = 707.13 [M + H+]

**Example 15: Preparation of $^{7m}$G$_{(2',3'Pivaloyl)}$pppA$_{(2'OMe)}$pG (15)**

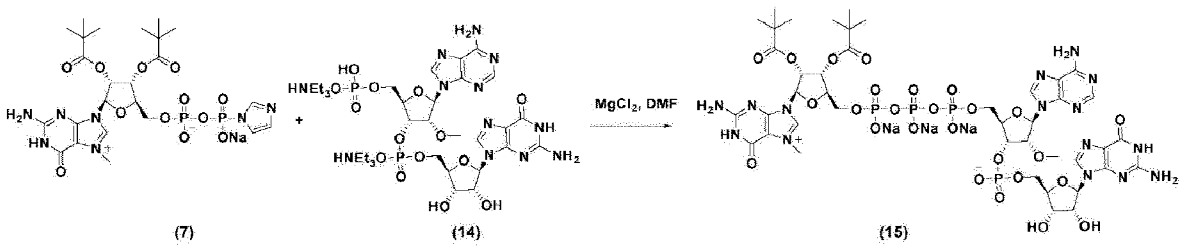


(7) (14) (15)

0.55 g of magnesium chloride was added to 27.5 mL of dimethylformamide and dissolved. 0.69 g (0.99 mmol) of Im-pp$^{7m}$G(2', 3'Pivaloyl) (7) and 0.5 g (0.55 mmol) of pA$_{(2'OMe)}$pG (14) were added to the reaction solution and stirred at room temperature for 24 hours. After completion of the reaction, 275 mL of 25 mM ethylenediaminetetraacetic acid aqueous solution was added dropwise to terminate the reaction, cooled to room temperature, and neutralized with 1M sodium bicarbonate aqueous solution. The reaction solution was purified using a C18 column (50 x 250 mm), distilled and vacuum dried. The dried solid was dissolved in 2.5 ml distilled water and then added to a solution of 299 mg (2.44 mmol) sodium perchlorate dissolved in 15 mL acetone, cooled to 4 °C, the generated crystal was filtered off, washed with cold acetone and vacuum dried to obtain 0.46 g (60.0 % yield) of the sodium salt (15) of the target compound.

$^1$H NMR (400 MHz, D$_2$O, 25°C): δ= 8.20 (s, 1H), 7.91 (s, 1H), 7.75 (s, 1H), 5.75 ~ 5.72 (m, 2H), 5.65 (m, 1H), 5.33 (m, 1H), 5.24 (m, 1H), 4.60 (m, 1H), 4.29 ~ 3.96 (m, 12H), 3.91 (m, 3H), 3.26 (m, 3H), 1.02 (s, 9H), 0.90 (s, 9H); $^{31}$P NMR (162 MHz, D$_2$O, 25°C): δ= -0.32 (s, 1P), -11.01 ~ -11.22 (dd, 2P), -22.26 (t, 1P). LC-MS (ESI, m/z) = 1314.27 [M + H$^+$].

**Example 16: Preparation of $^{7m}G_{(2'\ or\ 3'monopivaloyl)}pppA_{(2'OMe)}pG$ (16)**

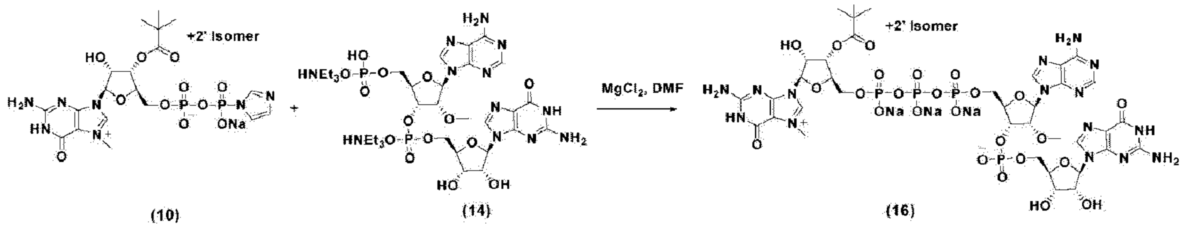

(10) (14) (16)

Reaction was carried out with the same method as Example 15, except that Im-pp$^{7m}G_{(2'\ or\ 3'monopivaloyl)}$ 0.61g (0.99 mmol) (10) of Example 10 was used as the starting substance instead of Im-pp$^{7m}$G(2',3'Pivaloyl) (7) of Example 15, and the title reaction product (mixture in molar ratio of 2'-monopivaloyl compound:3'-monopivaloyl compound = 0.65±0.05:1 (under water conditions at 25 °C±5, pH 1.0 to 8.0) (see FIG. 3) )) (16) 0.5 g (yield 70 %) was prepared by separating, purifying and distilling using a C18 column (50 × 250 mm) and changing the salt.

$^1$H NMR (400 MHz, $D_2O$, 25°C): δ= 8.30 (d, 1.65H, 2'-monopivaloyl and 3'-monopivaloyl compounds, overlapped), 8.00 (d, 1.65H, 2'-monopivaloyl and 3'-monopivaloyl compounds, overlapped), 7.85 (d, 1.65H, 2'-monopivaloyl and 3'-monopivaloyl compounds, overlapped), 5.88 ~ 5.73 (m, 4.95H, 2'-monopivaloyl and 3'-monopivaloyl compounds, overlapped), 5.30 (m, 0.65H, 2'H from 2'- monopivaloyl compound), 5.19 (m, 1H, 2'H from 3'-monopivaloyl compound), 4.86 (m, 1H, from 3'-monopivaloyl compound), 4.73 (m, 0.65H, from 2'-pivaloyl compound), 4.60 (t, 1H, 3'-pivaloyl compound), 4.53 (t, 0.65H, from 2'-pivaloyl compound), 4.43 ~ 4.13 (m, 19.80H, 2'-monopivaloyl and 3'-monopivaloyl compounds, overlapped), 4.00 (d, 4.95H, 2'-monopivaloyl and 3'-monopivaloyl compounds, overlapped), 3.37 (d, 4.95H, 2'-monopivaloyl and 3'-monopivaloyl compounds, overlapped), 1.16 (s, 9H, from 3'-monopivaloyl compound), 1.10 (s, 5.85H, from 2'-monopivaloyl compound); $^{31}$P NMR (162 MHz, $D_2O$, 25°C): δ= -0.35 (s, 1.65P, 2'-monopivaloyl and 3'-monopivaloyl compounds, overlapped), -10.85 (dd, 3.3P, 2'-monopivaloyl and 3'- monopivaloyl compounds, overlapped), -22.31 (t, 1.65P, 2'-monopivaloyl and 3'-monopivaloyl compound, overlapped). LC-MS (ESI, m/z) = 1230.22 [M + $H^+$].

**Experimental Example 1: In vitro transcription of luciferase mRNA by co-transcriptional capping**

For the mRNA synthesis reaction using the compounds prepared in Example 15 and Example 16 above, a transcription buffer was used, and the composition of the buffer was Tris-Cl buffer: spermidine:Triton X = 4:2:1 volume ratio. In the buffer, 50 pg/ml luciferase DNA transcription template, 10 mM ATP, 10 mM CTP, 10 mM UTP, 2 mM GTP, and 8 mM compound prepared in Example 15 or Example 16, 300 KU/ml T7 RNA polymerase, 2 KU/ml RNase inhibitor protein, 20 U/ml inorganic pyrophosphatase, 40 mM Tris-HCl (pH 8. 0), 50 mM magnesium chloride, and 10 mM dithiothreitol were added to prepare the transcription reaction mixture. In addition, as a positive control group, instead of the compounds of Example 15 or Example 16, $^{7m}G_{(3'OMe)}pppA_{(2'OMe)}pG$ (Cleancap AG(3'OMe) (TriLink catalog N-7413) was used to prepare the transcription reaction mixture. The transcription reaction mixture was reacted at 37 °C for 2 to 3 hours. Afterwards, 2KU/mL DNase I was added to the reactant to terminate the reaction, and the reaction was performed at 37 °C for 15 to 30 minutes. The reaction-terminated mRNA was purified using the RNeasy maxi kit (Quiagen catalog # 75162) or by reverse-phase high-performance liquid chromatography according to the manufacturer's instructions. The concentration of the purified mRNA sample was determined using a fluorometer instrument, and the total amount of mRNA was calculated by multiplying the concentration and reaction volume. Then, in order to derive the mRNA in vitro transcription (IVT) yield, the total amount of mRNA synthesized using each different cap analog was divided by the input L-DNA content to quantify the IVT yield value according to the cap analog. Specifically, an IVT yield value of 100 refers to that 100 μg mRNA was synthesized from 1 μg L-DNA. This may be calculated using [Equation 1] below. Then, the IVT yield value [Equation 1] of $^{7m}G_{(3'OMe)}pppA_{(2'OMe)}pG$ (Cleancap AG(3'OMe)), a representative third-generation cap analog, was designated as 100 %, The IVT yield value [Equation 1] by each example compound was divided by the yield value [Equation 1] of $^{7m}G_{(3'OMe)}pppA_{(2'OMe)}pG$ and multiplied by 100 to give a percentage ( %) of IVT yield by the compound of Example 15 or Example 16 over $^{7m}G_{(3'OMe)}pppA_{(2'OMe)}pG$ [see Equation 2].

[Equation 1]

IVT yield value = total amount of synthesized mRNA / amount of L-DNA input

IVT yield (%) = (IVT yield value for each example compound [Equation 1] / IVT yield value by $^{7m}G_{(3'OMe)pppA(2'OMe)pG}$ [Equation 1]) x 100 [Equation 2].

IVT yield values for each example and $^{7m}G_{(3'OMe)}pppA_{(2'OMe)}pG$ were independently repeated three times, and significance was verified through one-way ANOVA and Tukey's hoc tests. The results are shown in FIG. 4. It was confirmed that mRNAs were synthesized using the compound of either of Example 15 and Example 16 of the present disclosure with almost the same yield as the IVT yield by $^{7m}G_{(3'OMe)}pppA_{(2'OMe)}pG$, a conventional third-generation cap analog.

Experimental Example 2: Translation of luciferase mRNA in HeLa cells

The translational activity of the mRNAs generated by the IVT reactions of each Example 15, Example 16, and $^{7m}G_{(3'OMe)}pppA_{(2'OMe)}pG$ was evaluated by transfecting the corresponding mRNAs into the HeLa cell line. All mRNAs synthesized through IVT reaction before transfection were subjected to poly(A) tailing process. Specifically, IVT mRNA, whose mRNA synthesis reaction was terminated by adding DNase 1, was mixed with IVT mRNA:10x poly (A) polymerase reaction buffer:10 mM ATP solution:poly (A) polymerase enzyme (4KU/ml):nuclease-free water = 20:10:10:2:58 ratio (volume/volume), reacted at 37 °C for 30 minutes to 1 hour, and then incubated at 4 °C to terminate the reaction. The reaction terminated mRNA was purified using the RNeasy Maxi kit (Qiagen catalog # 75162) or reverse-phase high-performance liquid chromatography according to the manufacturer's instructions and used for transformation. HeLa cells were cultured in DMEM supplemented with 10 % FBS, 1 % penicillin/streptomycin at 37 °C in an atmosphere of 5 % $CO_2$. After plating $1\times10^4$ HeLa cells per well, the next day the cells were transfected with 100 ng of mRNA per well using transfection reagent (messengerMAX lipofectamine; Invitrogen catalog # LMRNA003): According to the recommendations of the transfection reagent manufacturer, tube A was prepared by diluting 0.3 μL of transfection reagent in 5 μL of composite medium (Opti-MEM; Life technologies) and incubating for 10 minutes at room temperature, and tube B was prepared by diluting 200 ng of prepared mRNA in 10 μL of Opti-MEM. The solutions in tube A and tube B were mixed and incubated for 5 minutes at room temperature. Afterwards, cells were transfected using the incubated mixed solution. Cells were harvested 6, 12, and 24 hours after transfection, and luciferase activity was measured using Renilla-Glo™ Luciferase Assay kit (Promega catalog #E2710) according to the manufacturer's recommendations: After removing the medium from the transfected cells, 50 μl phosphate buffer solution (PBS) was added. Afterwards, 50μl of Renilla-Glo™ Luciferase Assay reagent was added, mixed, and incubated for 10 minutes, and then luciferase activity was detected using Varioskan LUX Multimode Microplate Reader (Thermo Fisher) (FIG. 5). All luciferase activity measurement tests were each independently repeated 3 times, and significance was verified through one-way ANOVA and Tukey's hoc tests.

mRNA capped with compound (16) of Example 16 of the present disclosure showed higher luciferase activity than mRNA capped with conventional $^{7m}G_{(3'OMe)}pppA_{(2'OMe)}pG$ from 6 to 24 hours after transfection (FIG. 5). In addition, it may be inferred from the results 24 hours after transfection that the residual rate of protein expressed by mRNA capped with compound 16 was higher (FIG. 6).

According to the results in FIG. 5 and FIG. 6, the change of the 2' or 3' hydroxyl group of methyl guanosine into a non-selective monoester according to an embodiment of the present disclosure showed remarkable protein expression efficiency that was not expected from the conventional cap analog structure. This appears to be the result of introducing a reaction that does not selectively modify either the 2' or 3' hydroxyl groups of guanosine, but non-selectively, that is, spontaneously substitutes either the 2' or 3' hydroxyl groups, prepared in the monoester form, and introducing into the cap analog compound the property that the monoesterified cap analog compound maintains a dynamic equilibrium between the 2' or 3' structural isomers.

## Claims

1. A compound of Formula 1 below, or a pharmaceutically acceptable salt thereof:

[Formula 1]

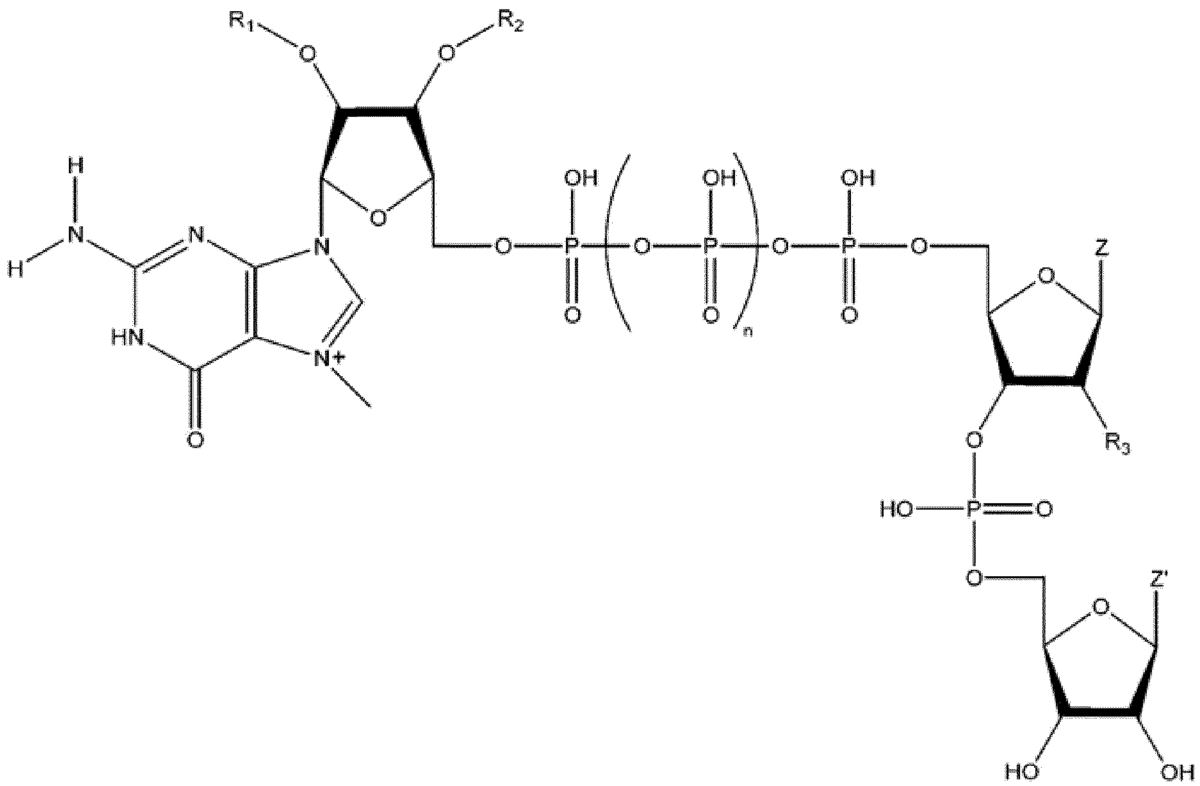

wherein,

n = 0, 1, or 2;
$R_1$ and $R_2$ are each independently H or C(=O)-R', wherein at least one of $R_1$ and $R_2$ is C(=O)-R', wherein R' is $C_1$ to $C_6$ alkyl or $C_1$ to $C_6$ alkoxy;
$R_3$ is a methoxy group;
Z and Z' are each independently a natural nitrogenous base.

**2.** The compound of claim 1, wherein the compound has the structure of Formula 1A below:

[Formula 1A]

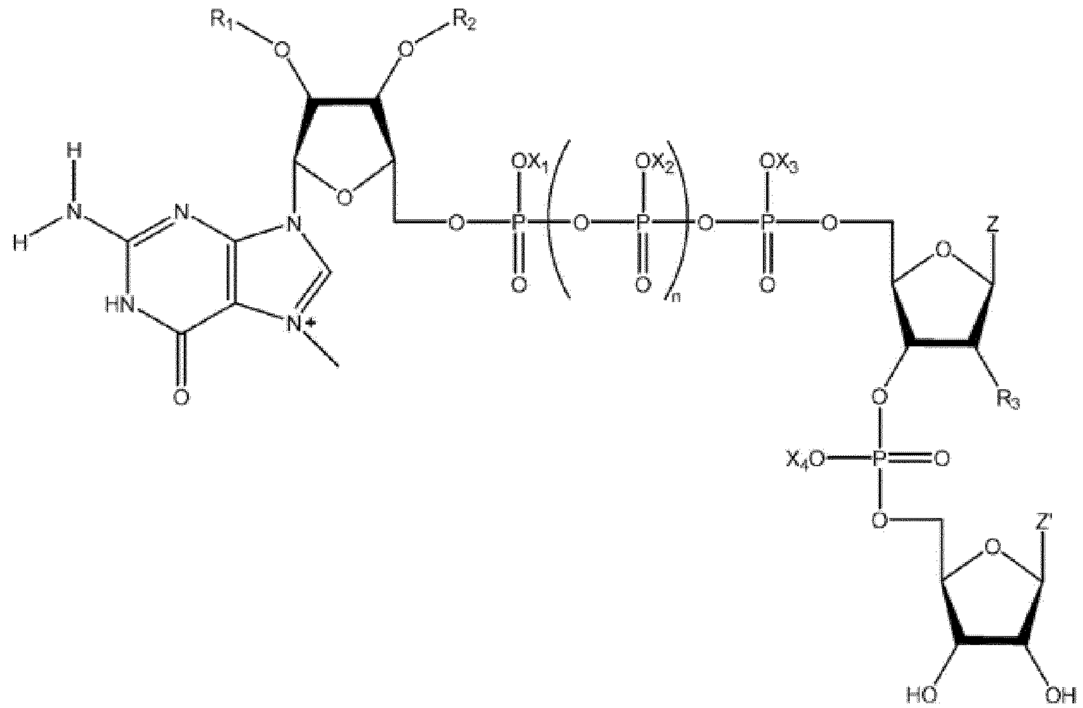

wherein,

n = 0, 1, or 2;

$X_1$, $X_2$, $X_3$, and $X_4$ are each independently absent, or are a monovalent cation, a divalent cation, or a combination thereof, wherein $X_1$, $X_2$, $X_3$, and $X_4$ are selected so that the compound of Formula 1A is electrically neutral;

$R_1$ and $R_2$ are each independently H or C(=O)-R', wherein at least one of $R_1$ and $R_2$ is C(=O)-R', wherein R' is $C_1$ to $C_6$ alkyl or $C_1$ to $C_6$ alkoxy;

$R_3$ is a methoxy group;

Z and Z' are each independently a natural nitrogenous base.

**3.** The compound of claim 2, wherein R' is $C_2$ to $C_6$ alkyl or $C_2$ to $C_6$ alkoxy.

**4.** The compound of claim 2, wherein the monovalent cation is selected from the group consisting of Na, Li, $NH_4^+$, and K, and the divalent cation is selected from the group consisting of $Mg^{2+}$, $Zn^{2+}$, and $Ca^{2+}$.

**5.** The compound of claim 2, wherein Z and Z' are each independently selected from the group consisting of guanine, adenine, cytosine, and uracil.

**6.** The compound of claim 1, which is selected from the group consisting of compounds of the following formulas, or a pharmaceutically acceptable salt thereof:

$^{7m}G_{(2'\ monopiva\iota oyl)}pppA_{(2'OMe)}pG$

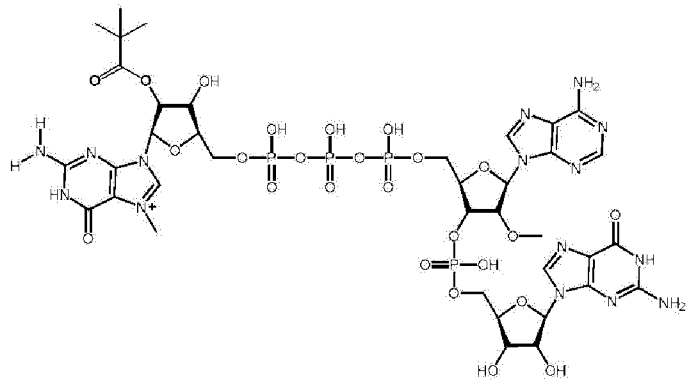

;

$^{7m}G_{(3'\ monopivaloyl)}pppA_{(2'OMe)}pG$

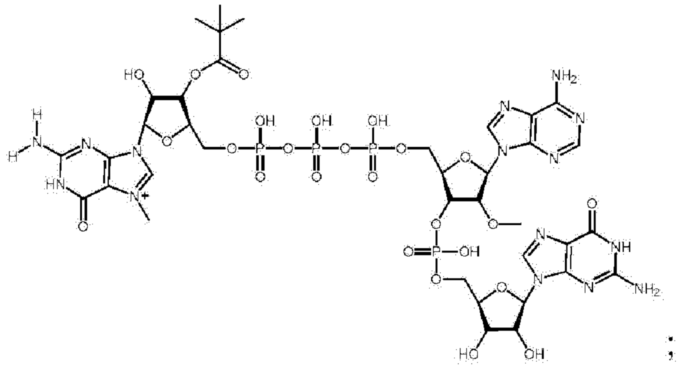

;

$^{7m}G_{(2',3'\ Pivaloyl)}pppA_{(2'OMe)}pG$

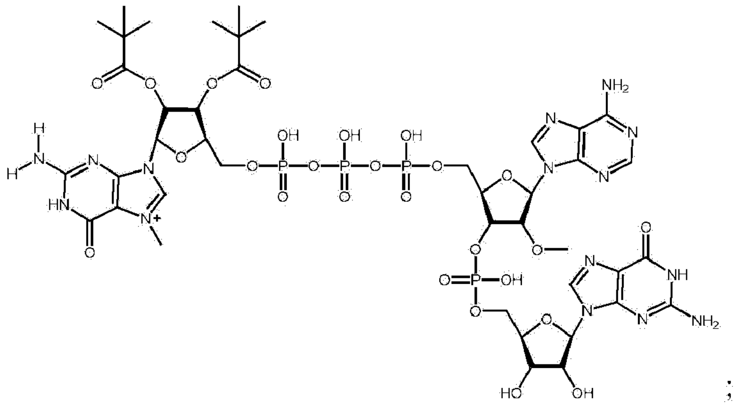

;

$^{7m}G_{(2'\ monoisobutyryl)}pppA_{(2'OMe)}pG$

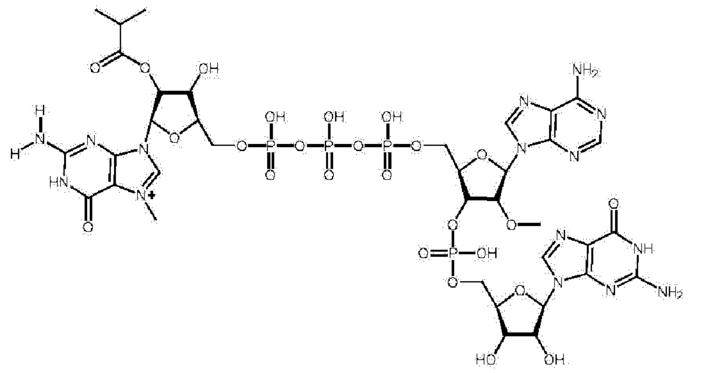

;

$^{7m}G_{(3'monoisobutyryl)}pppA_{(2'OMe)}pG$

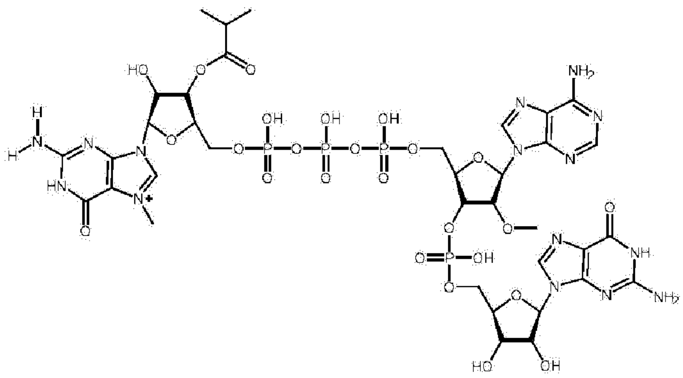

;

$^{7m}G_{(2',3'\ Isobutyryl)}pppA_{(2'OMe)}pG$

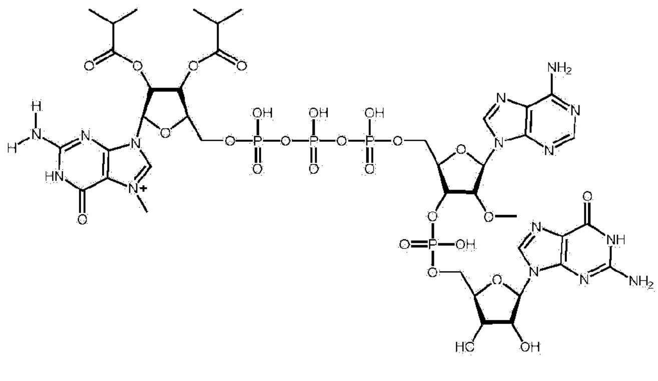

;

$^{7m}G_{(2'\ monoisopropoxy\ carbonyl)}pppA_{(2'OMe)}pG$

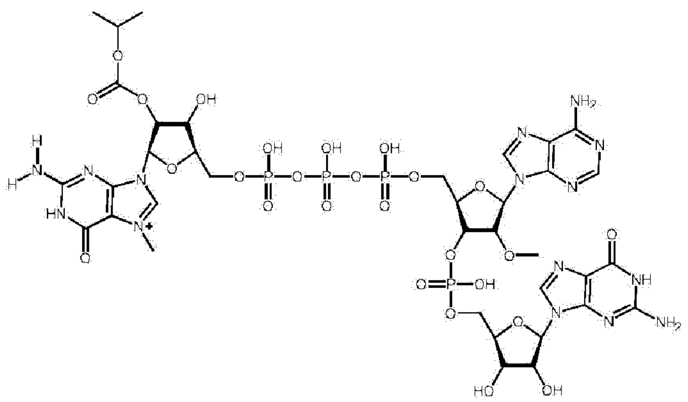

;

$^{7m}G_{(3'monoisopropoxy\ carbonyl)}pppA_{(2'OMe)}pG$

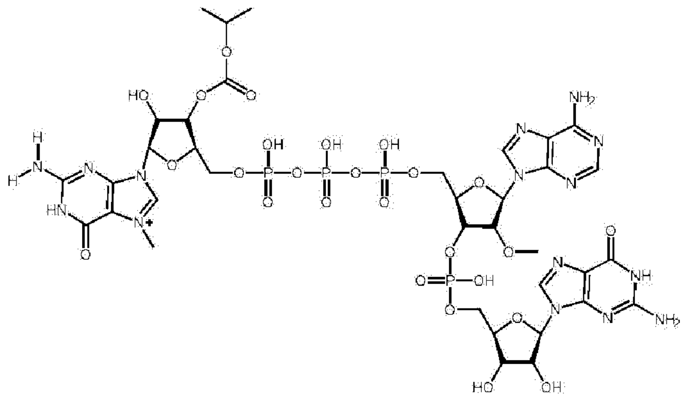

;

$^{7m}G_{(2',3'\ Isopropoxy\ carbonyl)}pppA_{(2'OMe)}pG$

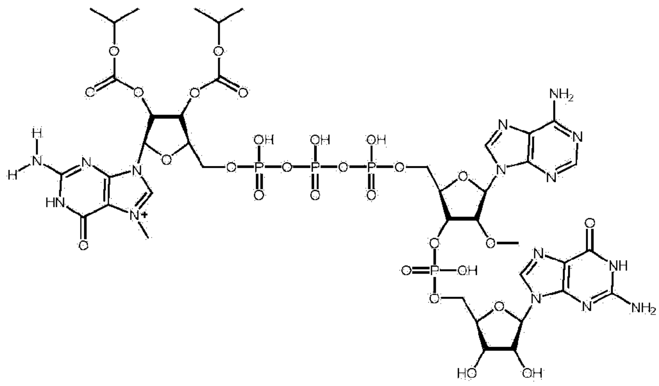

;

$^{7m}G_{(2'\ monoacetyl)}pppA_{(2'OMe)}pG$

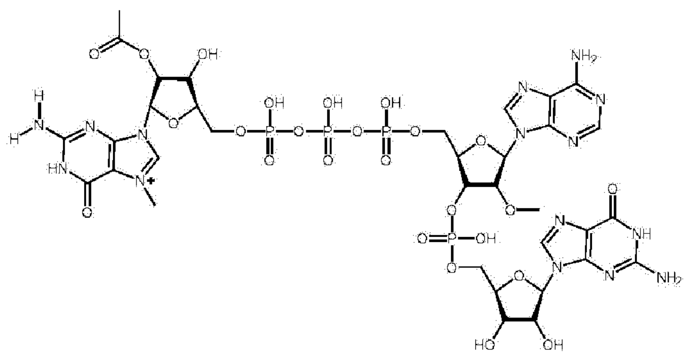

;

$^{7m}G_{(3'monoacetyl)}pppA_{(2'OMe)}pG$

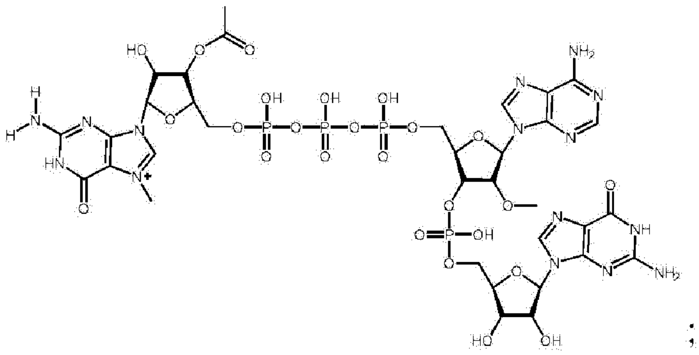

;

$^{7m}G_{(2',3'Acetyl)}pppA_{(2'OMe)}pG$

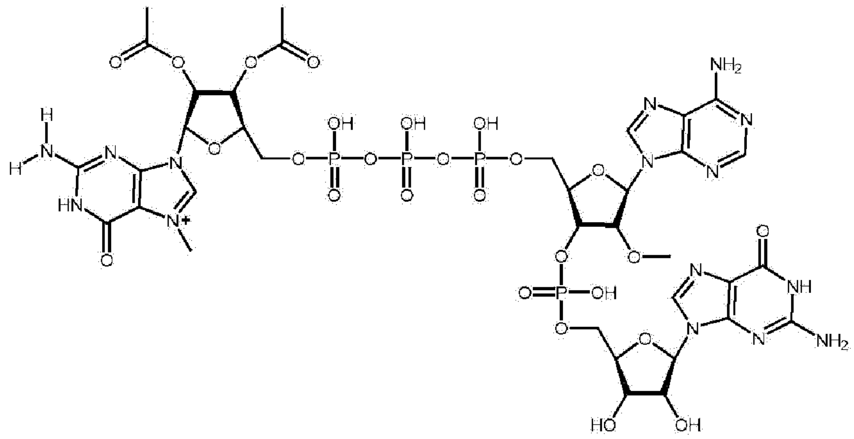

**7.** An aqueous composition comprising compounds of Formula 1A and Formula 1B below in a molar ratio of 0.65±0.05 : 1:

[Formula 1A]

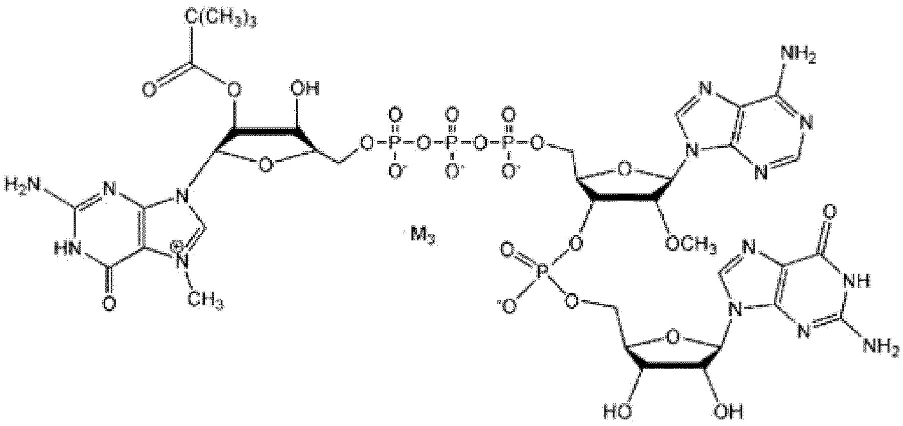

[Formula 1B]

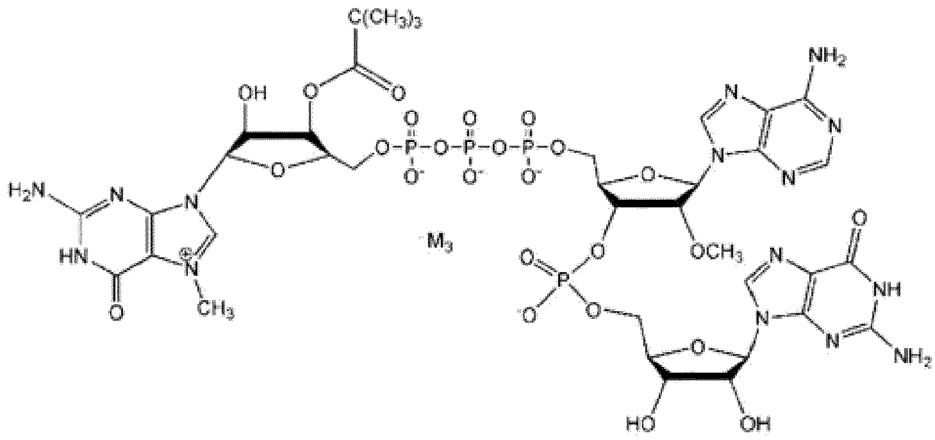

wherein, each M of $M_3$ is each independently not present, or is a monovalent cation selected from the group consisting of $Na^+$, Li+, $NH_4^+$, and K+, or a divalent cation selected from the group consisting of $Mg^{2+}$, $Zn^{2+}$, and $Ca^{2+}$, wherein $M_3$ is selected so that the compounds are electrically neutral.

**8.** The aqueous composition of claim 7, wherein each M of the $M_3$ is all $Na^+$.

**9.** The aqueous composition of claim 7, wherein pH of the aqueous composition is 1.0 to 8.0.

**10.** A cap analog, comprising a compound as defined in any one of claims 1 to 9.

**11.** An mRNA 5'-capped with a cap analog according to claim 10.

**12.** A composition or kit for preparing a 5' capped mRNA, comprising a cap analog according to claim 10.

**13.** A pharmaceutical composition for expressing a peptide or protein of interest, comprising an mRNA 5'-capped with a cap analog according to claim 10 and a pharmaceutically acceptable carrier.

**14.** A method of preparing a compound of formula 6 or a salt thereof, comprising steps of preparing a compound of formula 4 or a salt thereof by reacting a compound of Formula 2 below or a salt thereof with a compound of Formula 3 below under alkaline conditions in an aqueous phase; and

reacting the compound of Formula 4 or a salt thereof with a compound of Formula 5 under conditions of pH 1.5 to 4.5 *In situ:*

[Formula 2]

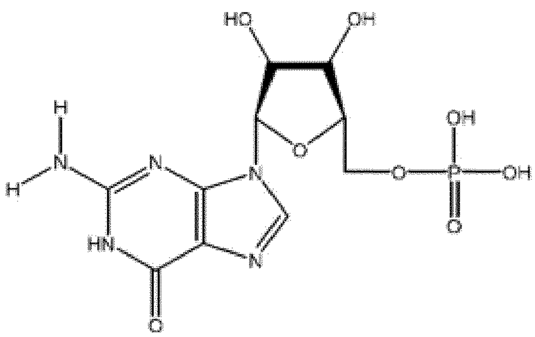

[Formula 3]

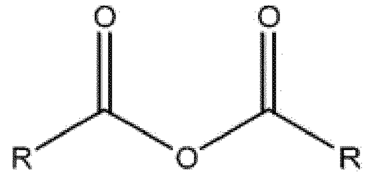

[Formula 4]

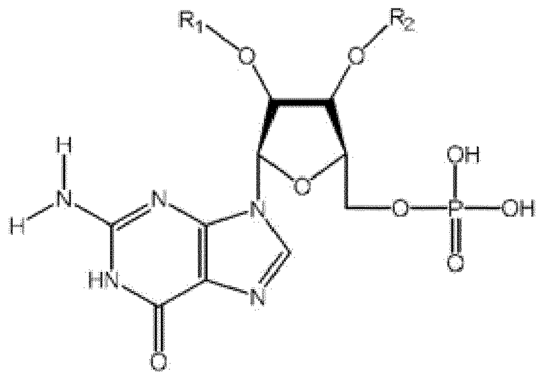

[Formula 5]

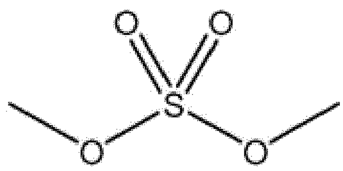

[Formula 6]

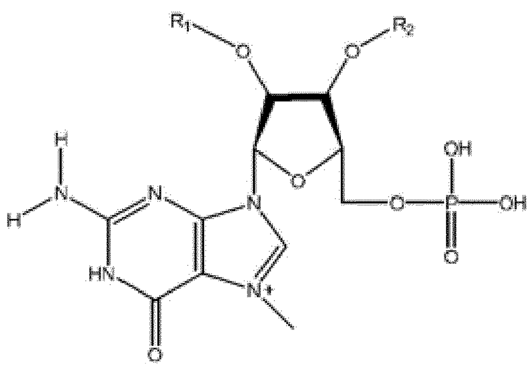

wherein in the above formulas, R refers to $C_1$ to $C_6$ alkyl or $C_1$ to $C_6$ alkoxy, and $R_1$ and $R_2$ are each independently H or C(=O)-R', wherein one of $R_1$ and $R_2$ is H and the other is C(=O)-R.

**15.** A method of preparing a compound of formula 8 or a salt thereof, comprising steps of preparing a compound of

Formula 7 or a salt thereof by reacting a compound of Formula 6 or a salt thereof with imidazole; and

reacting a compound of formula 7 or a salt thereof with zinc chloride and triethylammonium phosphate *In situ:*

[Formula 6]

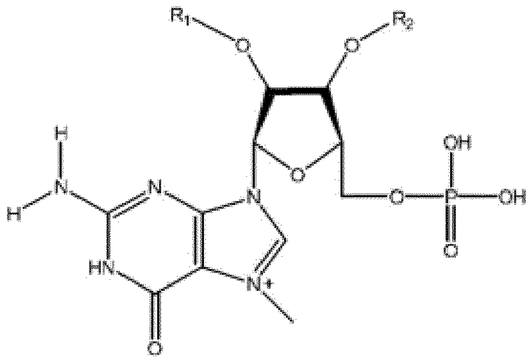

[Formula 7]

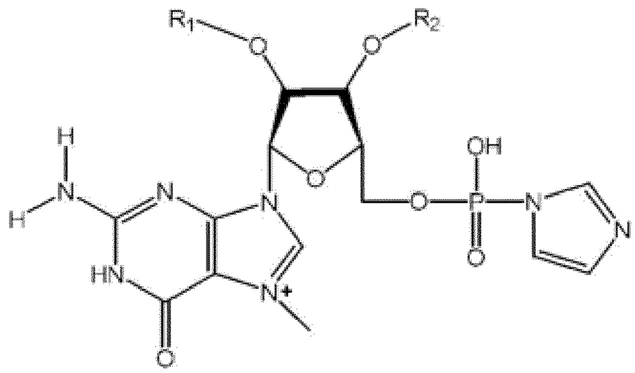

[Formula 8]

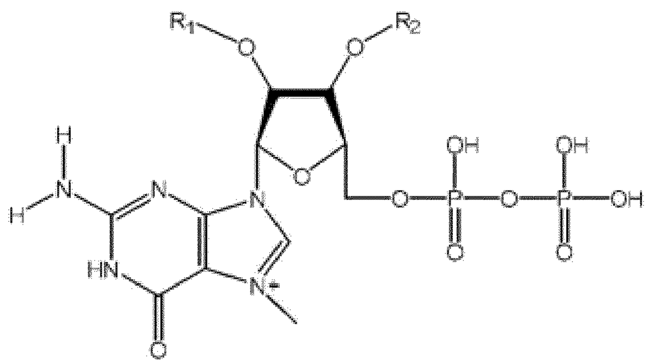

wherein in the above formula, R refers to $C_1$ to $C_6$ alkyl or $C_1$ to $C_6$ alkoxy, and $R_1$ and $R_2$ are each independently H or C(=O)-R, wherein one of $R_1$ and $R_2$ is H and the other is C(=O)-R.

FIG. 1

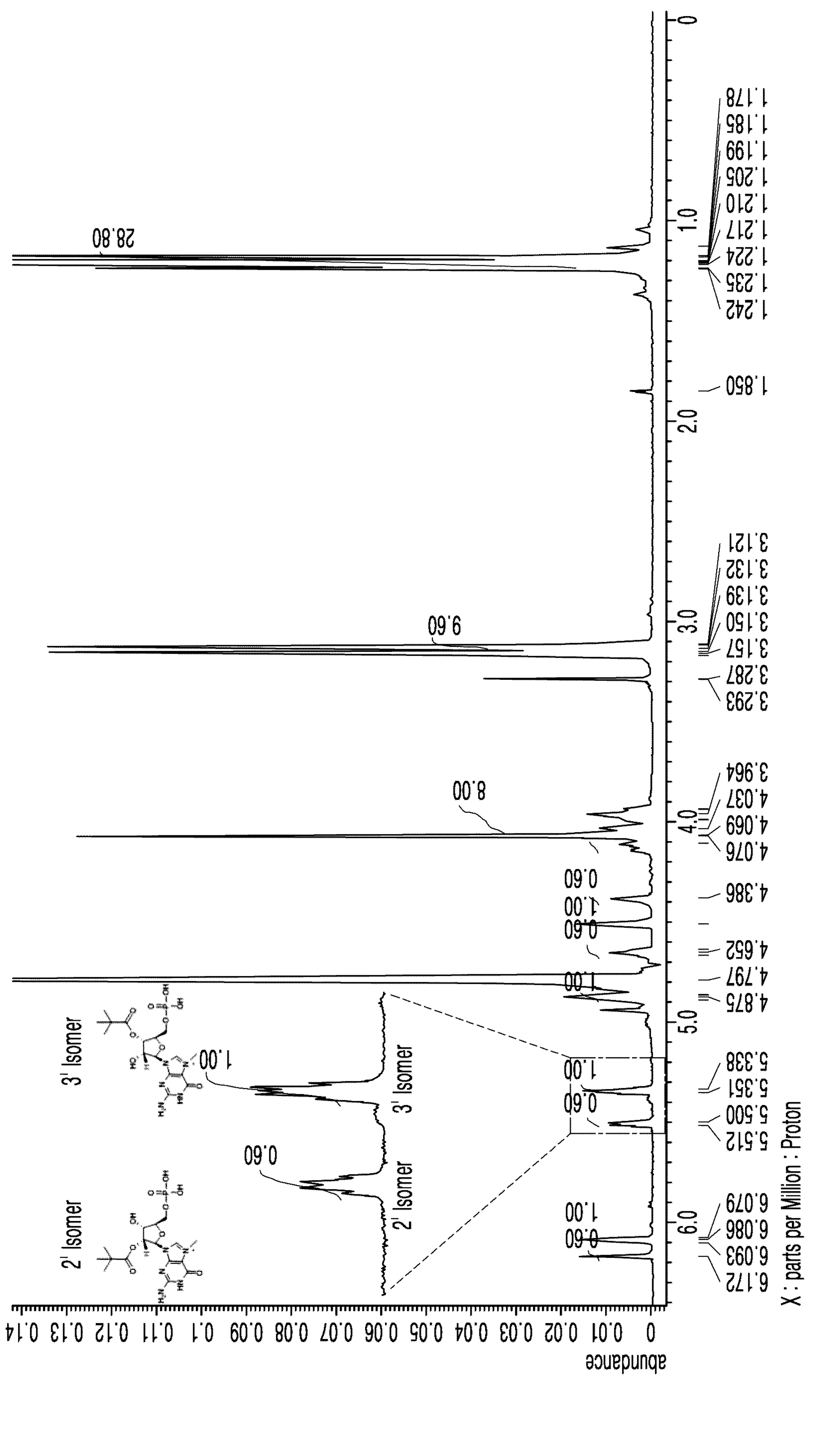
2' Isomer
3' Isomer
28.80
9.60
8.00
1.00
0.60
1.178
1.185
1.199
1.205
1.210
1.217
1.224
1.235
1.242
1.850
3.121
3.132
3.139
3.150
3.157
3.287
3.293
3.964
4.037
4.069
4.076
4.386
4.652
4.797
4.875
5.338
5.351
5.500
5.512
6.079
6.086
6.093
6.172
X : parts per Million : Proton
abundance

FIG. 2

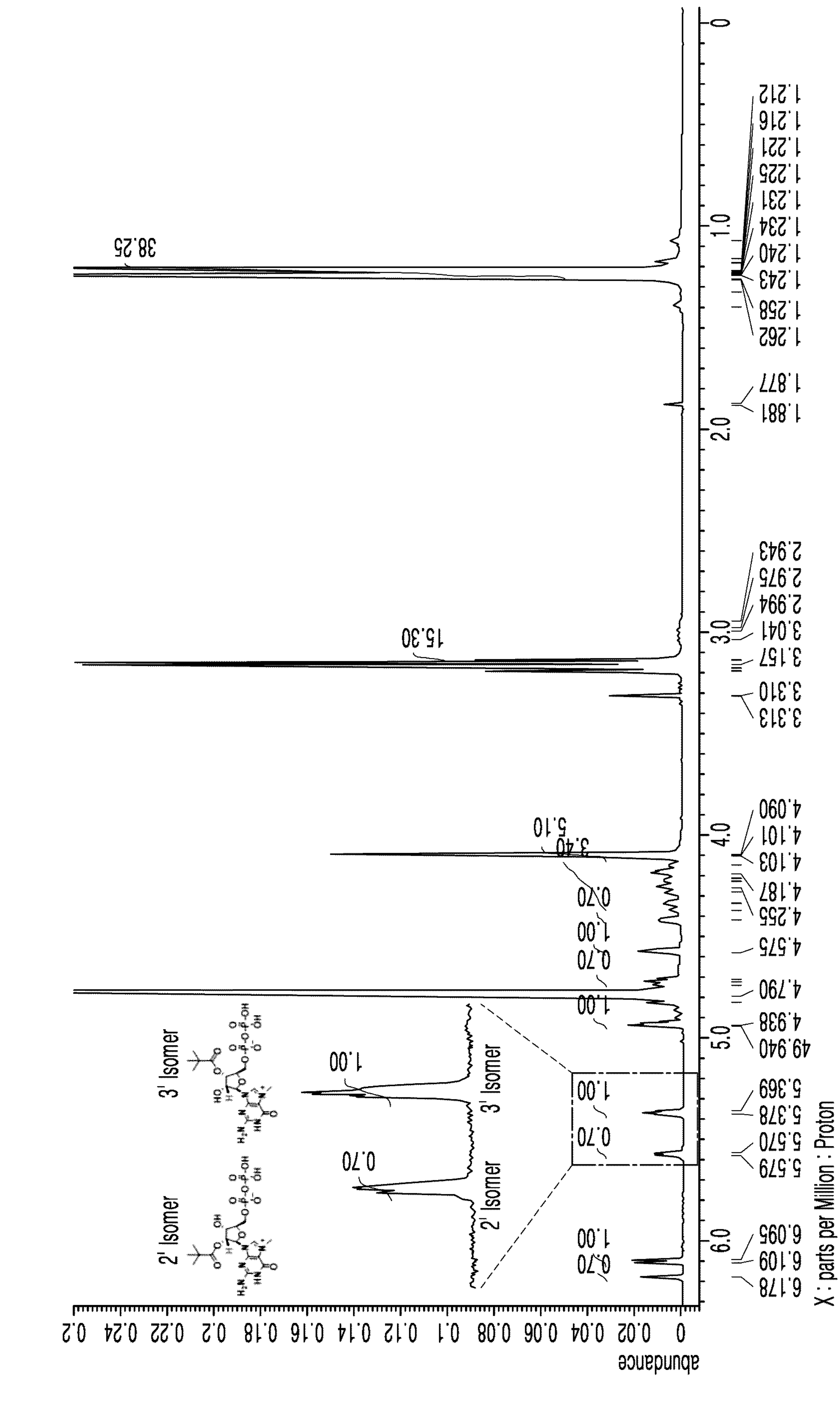

FIG. 3

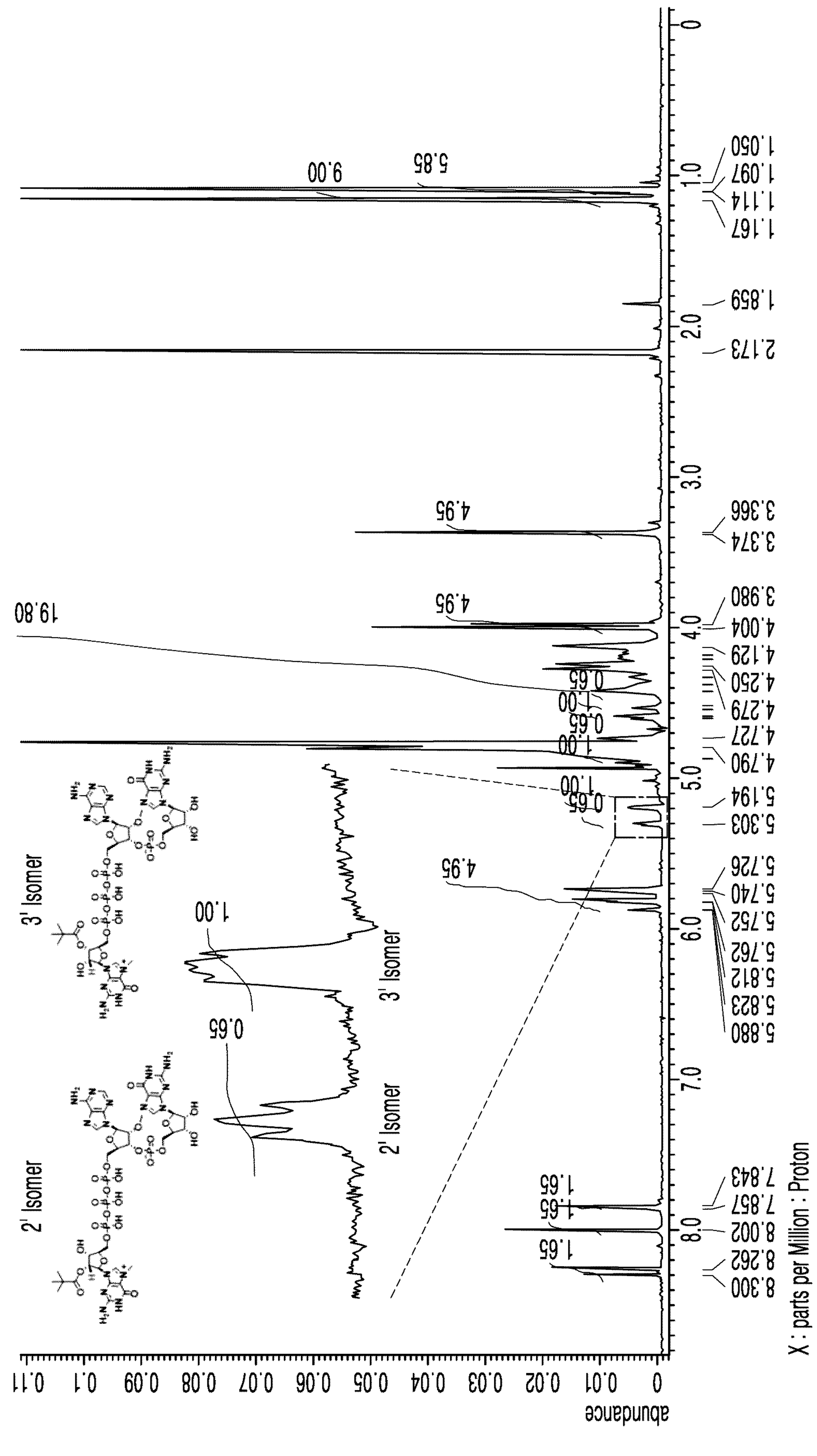
2' Isomer
3' Isomer
2' Isomer
3' Isomer
0.65
1.00
19.80
9.00
5.85
4.95
4.95
4.95
1.65
1.65
1.65
1.050
1.097
1.114
1.167
1.859
2.173
3.366
3.374
3.980
4.004
4.129
4.250
4.279
4.727
4.790
5.194
5.303
5.726
5.740
5.752
5.762
5.812
5.823
5.880
7.843
7.857
8.002
8.262
8.300
X : parts per Million : Proton
abundance

FIG. 4

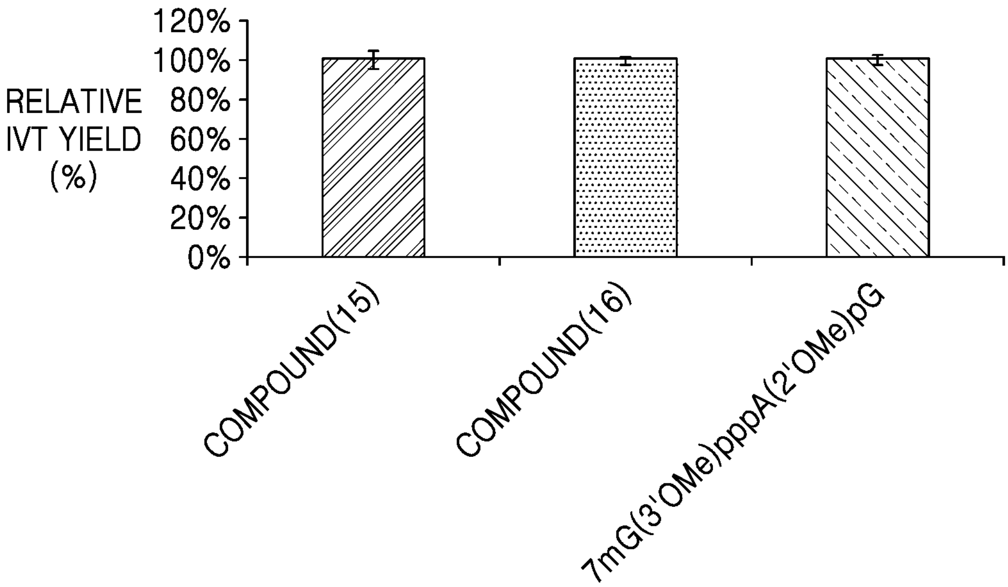


MEAN±STANDARD ERROR(n=3),***:P<0.05, NS:Not significant

FIG. 5

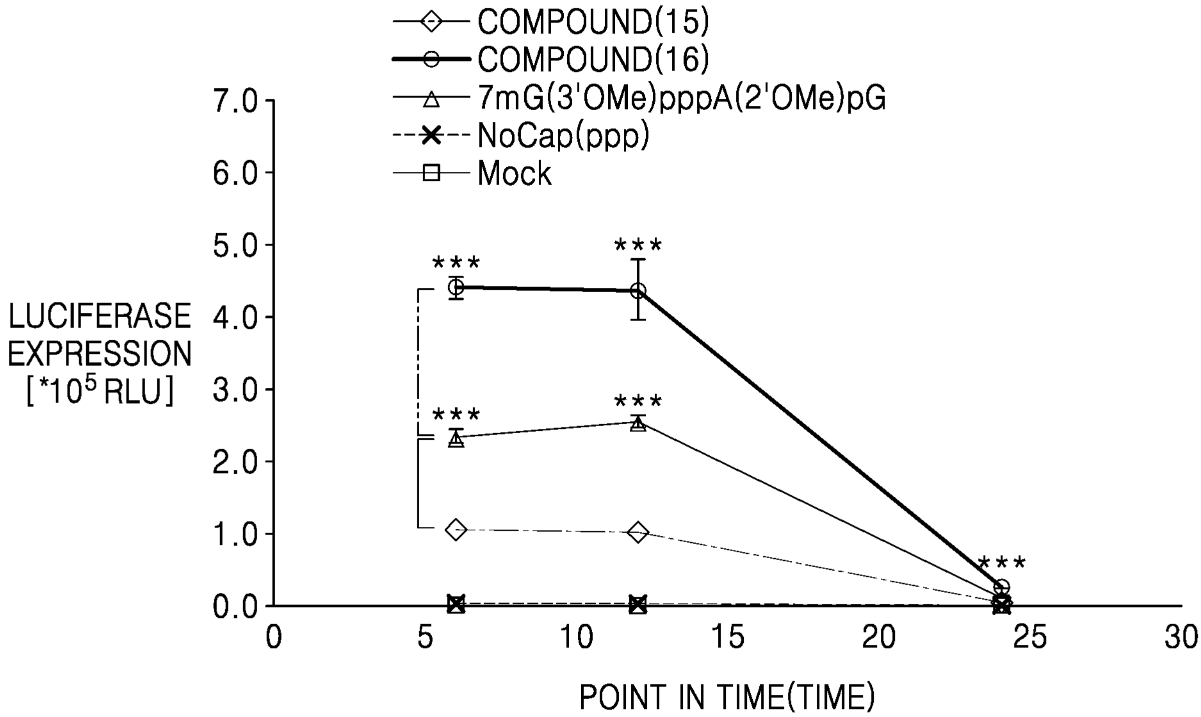


MEAN±STANDARD ERROR(n=3),***:P<0.05, NS:Not significant

FIG. 6

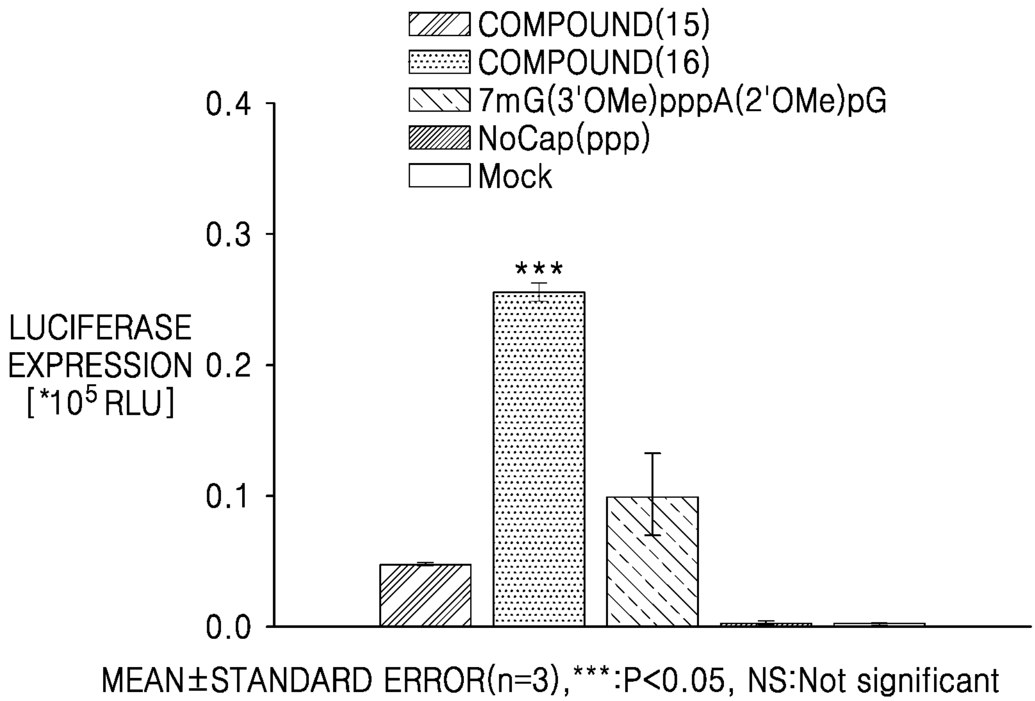
COMPOUND(15)
COMPOUND(16)
7mG(3'OMe)pppA(2'OMe)pG
NoCap(ppp)
Mock
0.4
0.3
0.2
0.1
0.0
***
LUCIFERASE EXPRESSION [*10^5 RLU]
MEAN±STANDARD ERROR(n=3),***:P<0.05, NS:Not significant

## INTERNATIONAL SEARCH REPORT

International application No.
**PCT/KR2022/013062**

**A. CLASSIFICATION OF SUBJECT MATTER**

**C07H 21/00**(2006.01)i; **C12N 15/113**(2010.01)i; **A61K 31/7088**(2006.01)i

According to International Patent Classification (IPC) or to both national classification and IPC

**B. FIELDS SEARCHED**

Minimum documentation searched (classification system followed by classification symbols)

C07H 21/00(2006.01); A61K 31/711(2006.01); A61K 9/127(2006.01); C07H 19/16(2006.01); C12N 15/11(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CasReact, Caplus), PubChem, Google & keywords: 구아닌(guanine), mRNA 캡 유사체(mRNA cap analog), 인산염(phosphate)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2018-0050409 A (TRILINK BIOTECHNOLOGIES, INC.) 14 May 2018 (2018-05-14) See claims 1 and 14-17; paragraphs [0057], [0177], [0190]-[0192] and [0232]-[0234]; and figures 1 and 10b. | 1-6,10-14 |
| A | | 7-9,15 |
| X | KORE, A. R. et al. Design and facile synthesis of new dinucleotide cap analog containing both 2` and 3'-OH modification on m7guanosine moiety. Nucleosides, nucleotides and nucleic acids. 2015, vol. 34, no. 9, pp. 611-619. See pages 612-613; and scheme 2. | 15 |
| Y | | 1-6,10-14 |
| Y | TRAPERO, A. et al. Covalent inactivation of mycobacterium thermoresistibile inosine-5′-monophosphate dehydrogenase (IMPDH). Bioorganic & medicinal chemistry letters. 2020, vol. 30, no. 2, article no. 126792, pp. 1-4. See page 2; and scheme 1. | 14 |
| A | US 2003-0194759 A1 (DARZYNKIEWIZ, E. et al.) 16 October 2003 (2003-10-16) See entire document. | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 December 2022** | **09 December 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

INTERNATIONAL SEARCH REPORT

International application No.
**PCT/KR2022/013062**

**C. DOCUMENTS CONSIDERED TO BE RELEVANT**

| Category* | Citation of document, with indication, where appropriate, of the relevant passages | Relevant to claim No. |
|---|---|---|
| A | US 2009-0324708 A1 (ALONSO, D. F. et al.) 31 December 2009 (2009-12-31) See entire document. | 1-15 |

Form PCT/ISA/210 (second sheet) (July 2022)

**INTERNATIONAL SEARCH REPORT**
**Information on patent family members**

International application No.
**PCT/KR2022/013062**

| Patent document cited in search report | Publication date (day/month/year) | Patent family member(s) | Publication date (day/month/year) |
|---|---|---|---|
| KR 10-2018-0050409 A | 14 May 2018 | AU 2016-328645 A1 | 19 April 2018 |
| | | AU 2016-328645 B2 | 13 May 2021 |
| | | AU 2016-328645 C1 | 28 October 2021 |
| | | AU 2021-206780 A1 | 12 August 2021 |
| | | CA 2999274 A1 | 30 March 2017 |
| | | CN 108366604 A | 03 August 2018 |
| | | CN 113584020 A | 02 November 2021 |
| | | DK 3352584 T3 | 12 July 2021 |
| | | EP 3352584 A1 | 01 August 2018 |
| | | EP 3352584 A4 | 27 March 2019 |
| | | EP 3352584 B1 | 12 May 2021 |
| | | EP 3906789 A1 | 10 November 2021 |
| | | EP 3954224 A1 | 16 February 2022 |
| | | EP 3954225 A1 | 16 February 2022 |
| | | ES 2879686 T3 | 22 November 2021 |
| | | HK 1255236 A1 | 09 August 2019 |
| | | HR P20211091 T1 | 15 October 2021 |
| | | HU E055458 T2 | 29 November 2021 |
| | | JP 2018-527015 A | 20 September 2018 |
| | | JP 2021-048864 A | 01 April 2021 |
| | | JP 2022-109973 A | 28 July 2022 |
| | | JP 6814997 B2 | 20 January 2021 |
| | | JP 7082174 B2 | 07 June 2022 |
| | | LT 3352584 T | 10 August 2021 |
| | | PL 3352584 T3 | 08 November 2021 |
| | | PT 3352584 T | 13 July 2021 |
| | | RS 62129 B1 | 31 August 2021 |
| | | SI 3352584 T1 | 30 September 2021 |
| | | US 10494399 B2 | 03 December 2019 |
| | | US 10519189 B2 | 31 December 2019 |
| | | US 10913768 B2 | 09 February 2021 |
| | | US 11414453 B2 | 16 August 2022 |
| | | US 2018-0273576 A1 | 27 September 2018 |
| | | US 2019-0144490 A1 | 16 May 2019 |
| | | US 2019-0270766 A1 | 05 September 2019 |
| | | US 2021-0261597 A1 | 26 August 2021 |
| | | US 2021-0371452 A1 | 02 December 2021 |
| | | US 2022-0289786 A1 | 15 September 2022 |
| | | WO 2017-053297 A1 | 30 March 2017 |
| US 2003-0194759 A1 | 16 October 2003 | US 2006-0252115 A1 | 09 November 2006 |
| | | US 7074596 B2 | 11 July 2006 |
| US 2009-0324708 A1 | 31 December 2009 | AR 058834 A1 | 27 February 2008 |
| | | EP 2089409 A1 | 19 August 2009 |
| | | WO 2008-055875 A1 | 15 May 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10913768 B
- US 7074596 B


### Non-patent literature cited in the description


- *RNA,* 2003, vol. 9, 1108-1122